(19) Europäisches Patentamt
European Patent Office
Office européen des brevets

(11) EP 0 563 013 B1

(12) EUROPÄISCHE PATENTSCHRIFT

(45) Veröffentlichungstag und Bekanntmachung des Hinweises auf die Patenterteilung:
**23.04.1997 Patentblatt 1997/17**

(51) Int Cl.[6]: **C08B 37/08**, A61K 7/48, C08J 5/18, C08B 37/00 // C08L5/08

(21) Anmeldenummer: **93810197.9**

(22) Anmeldetag: **18.03.1993**

# (54) N-Substituierte Chitosanderivate, ein Verfahren zu deren Herstellung und deren Verwendung

N-substituted chitosane derivatives, process for preparing them and their use

Dérivés de chitosane substitués sur l'atome d'azote, leur procédé de préparation et leur utilisation

(84) Benannte Vertragsstaaten:
**AT BE CH DE FR GB IT LI NL SE**

(30) Priorität: **27.03.1992 CH 981/92**

(43) Veröffentlichungstag der Anmeldung:
**29.09.1993 Patentblatt 1993/39**

(73) Patentinhaber: **Ciba Specialty Chemicals Holding Inc.**
**4057 Basel (CH)**

(72) Erfinder:
- **Lohmann, Dieter, Dr.**
  **CH-4142 Münchenstein (CH)**
- **Randell, Donald Richard, Dr.**
  **Stockport, Cheshire (GB)**

(56) Entgegenhaltungen:
**DE-A- 3 432 227**

- **CHEMICAL ABSTRACTS, vol. 82, no. 8, 24. Februar 1975, Columbus, Ohio, US; abstract no. 45565x, ANIKEEVA A. N. ET AL. 'Complex salts of chitosan or its derivatives' Seite 143 ;Spalte 45 ;**
- **DATABASE WPI Week 6600, Derwent Publications Ltd., London, GB; AN 28468F & SU-A-187 012 (LENINGRAD CHEM PHARM INST)**
- **CHEMICAL ABSTRACTS, vol. 67, no. 9, 28. August 1967, Columbus, Ohio, US; abstract no. 43395h, F.L.LUKNITSKII ET AL. 'Synthesis and reactions of beta-lactones.' Seite 4060 ;Spalte 43 ;**
- **EUR. POLYM. J. Bd. 25, Nr. 4 , 1989 Seiten 379 - 384 E. LOUBAKI ET AL. 'Modification chimique du Chitosane avec la delta-Gluconlactone, la beta-propiolactone et le glycidol'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft Chitosanderivate, deren Aminogruppen mit Hydroxacyl substituiert sind, das eine Carbonsäure-, Phosphonsäure- oder Sulfonsäuregruppe enthält oder deren Ester und Salze, ein Verfahren zu deren Herstellung durch die Umsetzung eines Chitosans mit einem 4- bis 6-gliedrigen Lacton oder einem 4-gliedrigen Sulton, das Sulfonsäureester-, Phosphonsäureester- oder Carbonsäureester- oder $CCl_3$-Gruppen enthält, und $CCl_3$-Gruppen anschliessend hydrolysiert, Estergruppen gegebenenfalls in Säuregruppen und Säuregruppen anschliessend gegebenenfalls in Salze überführt, sowie deren Verwendung zur Verhinderung von Haftung und/oder Bildung von festen Belägen auf anorganischen oder organischen Substraten, sowie deren Verwendung als Befeuchtungsmittel der Haut und Schleimhäute.

In der DE-A-2 222 733 sind Chitosanderivate beschrieben, deren Aminogruppen mit Carboxyalkyl substituiert sind. Sie werden durch die Umsetzung von Chitosan mit cyclischen Anhydriden wie zum Beispiel Bernsteinsäureanhydrid hergestellt. Hydroxylsubstituierte Anhydride werden nicht erwähnt und bei der Beschreibung des Herstellungsverfahrens sogar ausgeschlossen. Diese Chitosanderivate können als Sesquestriermittel, Detergentien und als Zusätze für kosmetische Mittel oder pharmazeutische Präparate verwendet werden.

In der DE-A-3 432 227 werden Sulfopropylderivate des Chitosans beschrieben, die durch die Umsetzung von Chitosan mit 1,3-Propansulton erhalten werden, und die als Zusätze zu kosmetischen Mitteln verwendet werden.

K. Kurita et al. beschreiben im Polymer Journal 22, No. 5, Seiten 429 bis 434(1990) die Umsetzung von Chitosan mit γ-Butyrolacton zur Herstellung γ-hydroxybutanoyl substituierter Chitosanderivate. E. Loubaki et al. beschreiben im Eur. Polym. J, 25, No. 4, Seiten 379 bis 384 (1989) eine ähnliche Modifizierung des Chitosans mit β-Propiolacton und δ-Gluconolacton.

Chitosanderivate, deren N-Substituent Hydroxylgruppen und saure Gruppen enthält, und die dadurch zur Bildung von Salzen fähig sind, sind noch nicht bekannt. Es wurde nun gefunden, dass man solche Chitosanderivate in guten Ausbeuten und guter Qualität erhält, wenn man anstelle von reaktionsträgen Lactonen solche mit einer aktivierenden Gruppe in α-Stellung zum Ringsauerstoff verwendet. Sie eignen sich in vielen Anwendungen als Ersatz für Hyaluronsäure.

Ein Gegenstand der Erfindung sind polymere Chitosanderivate mit Strukturelementen der Formeln I, II und III in statistischer Verteilung,

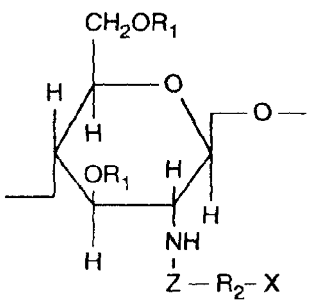
(I)

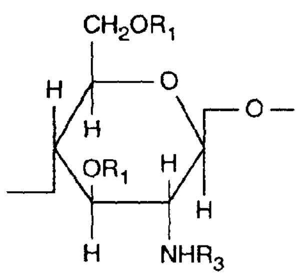
(II)

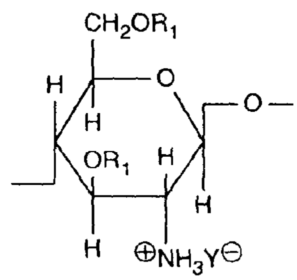
(III),

worin die $R_1$ unabhängig voneinander für H oder den Rest $-Z-R_2-X$ stehen, $R_3$ H oder Acetyl darstellt, Y das Anion $O-Z-R_2-X$ bedeutet, und

(a) Z für -CO- oder $-SO_2-$ steht, X $-CO_2H$, $-CH_2CO_2H$ oder $-CH_2PO(OH)_2$ bedeutet und $R_2$ $-CHR_4CR_5(OH)-$ darstellt, $R_4$ -H, -OH, $C_1$-$C_4$-A-koxy oder $C_1$-$C_4$-Alkyl ist, und $R_5$ H oder $C_1$-$C_4$-Alkyl bedeutet, oder
(b) Z für -CO- steht, X $-CO_2H$ bedeutet und $R_2$ $R_2-CHR_6-CHR_7-CH(OH)-$ oder $-CHR_8-CHR_9-CHR_{10}-CH(OH)-$ darstellt, $R_6$, $R_7$, $R_8$ und $R_{10}$ unahängig voneinander -H, -OH, $C_1$-$C_4$-Alkyl oder $C_1$-$C_4$-Alkoxy und $R_9$ -H, -OH, $C_1$-$C_4$-Alkyl, $C_1$-$C_4$-Alkoxy oder $-CO_2H$ bedeuten,

sowie deren Ester und Salze, wobei das Chitosanderivat insgesamt mindestens 2 Strukturelemente enthält und bezogen auf ein Mol des Chitosanderivats 30 bis 100 Mol-% Strukturelemente der Formel I, 60 bis 0 Mol-% Strukturelemente der Formel II und 30 bis 0 Mol-% Strukturelemente der Formel m enthalten und sich die Molprozente zu 100 % addieren.

In einer bevorzugten Ausführungsform enthält das Chitosanderivat insgesamt mindestens 4 Strukturelemente, vorzugsweise 4 bis 50, besonders bevorzugt 6 bis 30 Strukturelemente. Polymere Chitosanderivate können zum Beispiel bis zu 10 000, vorzugsweise bis zu 8000 und besonders bevorzugt bis zu 5000 Strukturelemente enthalten.

In einer bevorzugten Ausführungsform enthält das Chitosanderivat 50 bis 100 Mol-% Strukturelemente der Formel I, 50 bis 0 Mol-% Strukturelemente der Formel II und 20 bis 0 Mol-% Strukturelemente der Formel III, wobei sich die Molprozente zu 100 % addieren. Besonders bevorzugt enthält das Chitosanderivat 60 bis 100 Mol-% Strukturelemente der Formel I, 40 bis 0 Mol-% Strukturelemente der Formel II und 10 bis 0 Mol-% Strukturelemente der Formel III, wobei sich die Molprozente zu 100 % addieren.

In den Strukturelementen I, II und III steht (a) Z bevorzugt für -CO- und X bevorzugt für $-CO_2H$ oder $-CH_2PO(OH)_2$ oder Z für $-SO_2-$ und X für $-CO_2H$.

Wenn $R_4$ bis $R_{10}$ Alkyl bedeuten, so kann es sich um Methyl, Ethyl, n- oder i-Propyl oder n-, i- oder t-Butyl handeln. Bevorzugt bedeuten $R_4$ bis $R_{10}$ als Alkyl Methyl oder Ethyl.

Wenn $R_4$ bis $R_{10}$ Alkoxy bedeuten, so kann es sich um Methoxy, Ethoxy, Propoxy oder Butoxy handeln. Bevorzugt bedeuten $R_4$ bis $R_{10}$ als Alkoxy Methoxy.

Bevorzugt stellen $R_4$ und $R_6$ bis $R_{10}$ H, OH, Methyl oder Methoxy dar und $R_5$ H oder Methyl.

Einige Beispiele für $R_2$ sind $-CH_2CH(OH)-$, $-CH_2C(CH_3)(OH)-$, $-CH(OH)CH(OH)-$, $-CH(CH_3)CH(OH)-$, $-CH(OCH_3)CH(OH)-$, $-CH_2CH_2CH(OH)-$, $-CH(OH)CH_2CH(OH)-$, $-CH(OH)CH(OH)CH(OH)-$, $-CH(CH_3)CH_2CH(OH)-$, $-CH_2CH(OH)CH(OH)-$, $-CH(OCH_3)CH(OH)CH(OH)-$, $-CH_2CH(OCH_3)CH(OH)-$, $-CH_2CH_2CH_2CH(OH)-$, $-CH_2CH_2CH(OH)CH(OH)-$, $-CH_2CH(OH)CH(OH)CH(OH)-$, $-CH_2CH(COOH)CH_2CH(OH)-$, $-CH_2CH(OH)CH(CH_3)CH(OH)-$ und $-CH_2CH(OH)CH(OCH_3)CH(OH)-$. Besonders bevorzugt stellt $R_2$ $-CH_2CH(OH)-$ und $-CH_2C(CH_3)(OH)-$ dar.

Die Carboxyl- beziehungsweise Phosphonsäuregruppe kann verestert sein, zum Beispiel mit einem aliphatischen, cycloaliphatischen, araliphatischen oder aromatischen Alkohol, der 1 bis 30, bevorzugt 1 bis 20, und besonders bevorzugt 1 bis 12 C-Atome enthält. Beispiele sind Alkanole wie Methanol, Ethanol, Propanol, Butanol, Pentanol, Hexanol, Heptanol, Octanol, Nonanol, Decanol, Undecanol, Dodecanol, Tetradecanol, Octadecanol; Polyoxaalkanole wie Ethylenglykolmonomethylether oder -ethylether, Diethylenmonomethylether oder -ethylether, Oligoethylenglykole oder Oligopropylenglykole oder Mischpolymere davon mit insgesamt bis zu 20, bevorzugt bis zu 12 Monomereinheiten; Cycloalkanole wie Cyclopentanol und Cyclohexanol; Benzylalkohol und mit $C_1$-$C_{12}$-Alkyl substituierte Benzylalkohole; Phenol und mit $C_1$-$C_{12}$-Alkyl substituierte Phenole.

Die Carboxyl- beziehungsweise Phosphonsäuregruppen können auch als Salze ausgebildet sein. Es kann sich zum Beispiel um Metalle der Haupt- und Nebengruppen des Periodensystems der Elemente handeln, wie zum Beispiel die Metalle der dritten, vierten und fünften Hauptgruppe und den Nebengruppen des periodischen Systems der chemischen Elemente. Besonders geeignet sind Li, Na, K, Mg, Ca, Sr, Ba, B, Al, Ga, In, Sn, Pb, Sb, Bi, Cu, Ag, Au, Zn, Cd, Hg, Sc, Y, La, Ti, Zr, Hf, V, Nb, Ta, Cr, Mo, W, Fe, Co, Ni, Ru, Rh, Pd, Os, Ir, Pt und die Lanthanidenmetalle Ce, Pr, Nd, Pm, Sm, Eu, Gd, Tb, Dy, HO, Er, Tm, Yb und Lu. Bei Verwendung mehrwertiger Metalle können die entsprechenden Kationen als Vernetzer der Oligomer- beziehungsweise Polymerketten wirken. Bevorzugte Metalle sind die Alkali- und Erdalkalimetalle. Die Salze können auch als Aminsalze ausgebildet sein, zum Beispiel als Ammoniumsalze oder als Salze von primären, sekundären oder tertiären Aminen, die bevorzugt 1 bis 20 und besonders bevorzugt 1 bis 12 C-Atome enthalten, oder als Salze von Polyaminen mit primären, sekundären und/oder tertiären Amingruppen und bevorzugt 2 bis 20, besonders bevorzugt 2 bis 16 C-Atomen, oder als Salze eines Polymeren mit Aminogruppen in wiederkehrenden Strukturelementen.

Einige Beispiele für Amine sind Methylamin, Dimethylamin, Trimethylamin, Ethylamin, Diethylamin, Triethylamin, n-Propylamin, i-Propylamin, Di-n-propylamin, Di-i-propylamin, Tri-n-propylamin, Tri-i-propylamin, n-Butylamin, Di-n-butylamin, Tri-n-butylamin, Hexylamin, Dodecylamin, Octadecylamin, Eicosylamin, Morpholin, N-Methylmorpholin, Piperidin, N-Methylpiperidin, Anilin, N-Methylanilin, N-Dimethylanilin, Pyridin Pyrimidin, Ethanolamin, Diethanolamin und Triethanolamin.

Einige Beispiele für Polyamine sind Ethylendiamin, N,N'-Dimethylethylendiamin, Diethylentriamin, Triethylentetramin, 1,3-Diaminopropan, 1,3-Dimethylaminopropan, 1,4-Diaminobutan, Piperazin, Phenylendiamin, Naphthylendiamin, 4,4'-Diaminodiphenyl, 4,4'-Diaminodiphenylether, 4,4'-Diaminodiphenylthioether und 4,4'-Diaminodiphenylmethan.

Einige Beispiele für Polymere mit Aminogruppen sind Poly(aminosaccharide) wie Chitosan selbst und Polygalaktosamin, Albumin oder Polyethylenimin, niedermolekulare Polyamide mit endständigen Aminogruppen und aminoalkylierte Polyacrylsäure- oder Polymethacrylsäureamide.

Die erfindungsgemässen Chitosanderivate können in einfacher Weise durch die Umsetzung von Chitosan mit β-, γ- oder δ-Lactonen oder β-Sultonen erhalten werden, die eine veresterte Carboxylgruppe oder eine $CCl_3$-Gruppe in α-Stellung zum Ringsauerstoffatom enthalten, wobei die $CCl_3$-Gruppe anschliessend hydrolysiert und gegebenenfalls die Estergruppen in Säuregruppen und die Säuregruppen gegebenenfalls in Salze übergeführt werden.

Ein weiterer Gegenstand der Erfindung ist ein Verfahren zur Herstellung der erfindungsgemässen Chitosanderivate mit insgesamt mindestens 2 Strukturelementen der Formeln I, II und III in statistischer Verteilung, das dadurch gekennzeichnet ist, dass man ein Chitosan mit insgesamt mindestens 2 Strukturelementen der Formeln IV und V in statistischer Verteilung,

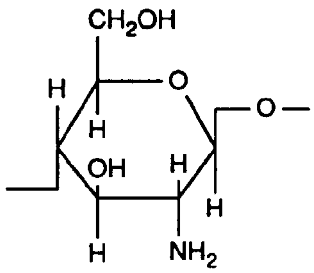 (IV), 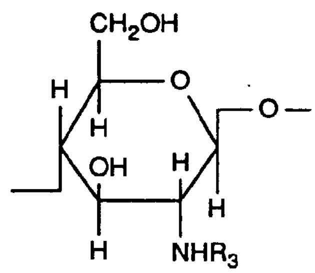 (V),

worin $R_3$ für Acetyl oder H steht, wobei das Chitosan 30 bis 100 Mol-% Strukturelemente der Formel IV und 70 bis 0 Mol-% der Strukturelemente der Formel V enthält, bezogen auf 1 Mol des Chitosans, in Gegenwart eines inerten Lösungsmittels mit mindestens 30 Mol.-% eines Lactons der Formeln VI, VII oder VIII, bezogen auf das Chitosan,

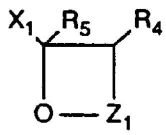 (VI), 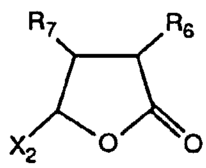 (VII), 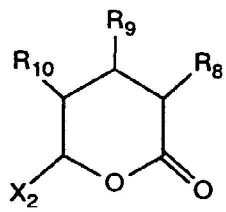 (VIII),

umsetzt, worin $R_4$, $R_5$, $R_6$, $R_7$, $R_8$, $R_9$ und $R_{10}$ die zuvor angegebenen Bedeutungen haben,

$Z_1$ für =CO oder =$SO_2$ steht, $X_1$-$CCl_3$, -$CO_2R_{11}$, -$CH_2CO_2R_{11}$ oder -$CH_2PO(OR_{11})_2$ bedeutet, $X_2$-$CO_2R_{11}$ darstellt, und $R_{11}$ der um die Hydroxylgruppe verminderte Rest eines Alkohols mit 1 bis 20 C-Atomen bedeutet, und die erhaltenen Chitosanderivate, worin $X_1$ für -$CCl_3$ steht, unter alkalischen Bedingungen unter Bildung der entsprechenden Carbonsäuresalze hydrolysiert, die Carbonsäuresalze und Ester gegebenenfalls in die entsprechenden Säuren überführt und die Säuren gegebenenfalls in Salze überführt. Die Carbonsäuresalze können durch Einstellung des pH-Wertes der Reaktionsmischung in die entsprechenden erfindungsgemässen Säuren übergeführt und auch als solche isoliert werden.

Das erfindungsgemässe Verfahren ermöglicht überraschend erstmals die Herstellung von N-acylierten Chitosanen mit Säure- und Hydroxygruppen in der N-Acylgruppe. Besonders vorteilhaft ist hierbei die Verwendung der $CCl_3$-Gruppen enthaltenden β-Lactone und -Sultone, da diese $CCl_3$-Gruppen in einfacher Weise in die Carboxylgruppe übergeführt werden können. Die Reaktion führt unter milden Reaktionsbedingungen zu hohen chemischen Umsätzen, wobei man sogar vollständige Substitutionen an der $NH_2$-Gruppe des Chitosans erzielen kann und Nebenreaktionen weitgehend vermieden werden können.

Ein weiterer Gegenstand der Erfindung sind die als Zwischenprodukte verwendeten Chitosanderivate mit Strukturelementen der Formeln IX und X in statistischer Verteilung,

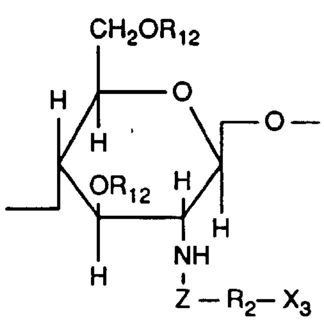 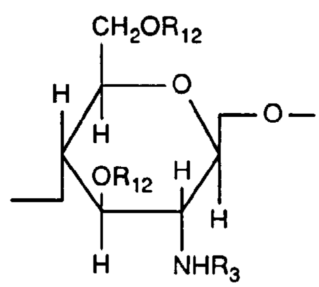

(IX) (X)

worin die $R_{12}$ unabhängig voneinander für H oder den Rest -Z-$R_2$-$X_3$ stehen, $R_3$ H oder Acetyl darstellt, Z für -CO- oder -$SO_2$- steht, $X_3$-$CCl_3$ bedeutet und $R_2$ -$CHR_4CR_5(OH)$- darstellt, $R_4$-H, -OH, $C_1$-$C_4$-Alkoxy oder $C_1$-$C_4$-Alkyl ist, und $R_5$ H oder $C_1$-$C_4$-Alkyl bedeutet, wobei das Chitosanderivat insgesamt mindestens 2 Strukturelemente enthält und

bezogen auf 1 Mol des Chitosanderivats 30 bis 100 Mol.-% Strukturelemente der Formel IX und 70 bis 0 Mol.-% Strukturelemente der Formel X enthält.

$R_{12}$ steht bevorzugt für H, und $X_3$ bedeutet bevorzugt $-CCl_3$. Z stellt bevorzugt -CO- dar. Für $R_2$, $R_4$ und $R_5$ gelten die zuvor angegebenen Bevorzugungen. Besonders bevorzugt stellt $R_2$ die Reste $-CH_2CH(OH)-$ und $CH_2C(CH_3)(OH)-$ dar. Für die Anzahl der Strukturelemente und den Gehalt an Strukturelementen gelten die zuvor für die erfindungsgemässen Chitosansäuren, -säureester und -säuresalze angegebenen Bevorzugungen.

Es hat sich als zweckmässig erwiesen, das käufliche Chitosan vor der Umsetzung zu aktivieren, indem man das Chitosan zum Beispiel in verdünnter Essigsäure löst, ungelöste Anteile abfiltriert und dann mit verdünnten wässrigen Basen, zum Beispiel NaOH bis zu einem pH-Wert von etwa 8-9 versetzt und so das Chitosan wieder ausgefällt. Man entfernt die Salze nach bekannten Methoden, zum Beispiel durch Auswaschen oder Dialyse. Das Produkt kann dann durch wiederholtes Zentrifugieren mit einem Nichtlösungsmittel, zum Beispiel Ethern wie Dioxan, entwässert werden. Man erhält so Produkte mit Wassergehalten von etwa 3 bis 10 Gew.-%, die stark gequollen sind und eine hohe Reaktionsfähigkeit aufweisen. Je nach Reaktionsbedingungen und dem Wassergehalt des verwendeten Chitosans können bei der Umsetzung durch Hydrolyse Hydroxycarbonsäuren gebildet werden, die durch Salzbildung zu Chitosanderivaten mit Strukturelementen der Formel III führen. Diese Reaktion kann aber durch milde Bedingungen vollständig unterdrückt werden, so dass auch Chitosanderivate ohne Strukturelemente der Formel III zugänglich sind. Bei drastischen Reaktionsbedingungen, höheren Wassergehalten beziehungsweise Acetylgruppengehalten des Chitosans können als Endprodukte hochquellbare Gele erhalten werden, die leicht vernetzt sind.

Bei der Umsetzung kann auch in geringem Umfang eine Reaktion der freien Hydroxylgruppen mit den Lactonen stattfinden, was aber durch die Wahl der Reaktionsbedingungen weitgehend unterdrückt werden kann. Chitosanderivate, worin in den Strukturelementen der Formeln I, II und III $R_1$ für H steht, sind bevorzugt.

Der Anteil der Strukturelemente der Formel II hängt zum einen vom Gehalt an Acetylgruppen im Chitosan und zum anderen von der Reaktivität der verwendeten Lactone und dem erzielbaren Substitutionsgrad ab. Käufliche Chitosane können bis zu 70 Mol-% acetylhaltige Strukturelemente der Formel V enthalten.

Oligomere Chitosane sind zum Beispiel durch einen hydrolytischen Abbau der Polymeren zugänglich, zum Beispiel mit verdünnten Mineralsäuren wie Salzsäure. Die erhaltenen Oligomerengemische werden dann mit zum Beispiel NaOH neutralisiert und mittels Diafiltration über eine Membran von Salzen und niedermolekularen Anteilen befreit. Die erhaltenen Oligomerengemische können als solche verwendet oder zuvor in bekannter Weise fraktioniert werden. Oligomere Chitosane können auch durch eine Deacetylierung von Chitosanoligomeren erhalten werden, zum Beispiel deacetylierte Chitobiose oder Chitohexaose, die auch kommerziell erhältlich sind.

Die erfindungsgemäss zu verwendenden Lactone sind bekannt, teilweise käuflich oder nach bekannten Verfahren herstellbar.

Das erfindungsgemässe Verfahren kann zum Beispiel so durchgeführt werden dass man das Chitosan in einem Lösungsmittel löst oder suspendiert, und dann in eine Lösung des Lactons einträgt, wobei diese Stufe vorzugsweise bei Raumtemperatur durchgeführt wird. Danach rührt man das Reaktionsgemisch bei gegebenenfalls erhöhten Temperaturen weiter und lässt ausreagieren. Die Reaktion kann bei Temperaturen von 0 bis 150 °C, bevorzugt 10 bis 120 °C und besonders bevorzugt 20 bis 100 °C durchgeführt werden.

Die Reaktion wird vorteilhaft unter Ausschluss von Feuchtigkeit, zum Beispiel Luftfeuchtigkeit oder einem Wassergehalt in Lösungsmitteln, durchgeführt. Im allgemeinen führt man das Verfahren zweckmässig unter einer trockenen Inertgasatmosphäre durch, zum Beispiel aus Edelgasen (Helium oder Argon) oder Stickstoff.

Geeignete Lösungsmittel sind zum Beispiel polare aprotische Lösungsmittel, die alleine oder in Mischung von mindestens zwei Lösungsmitteln verwendet werden können und das verwendete Chitosan lösen oder quellen. Die Lösungsmittel können auch als Suspensionsmittel eingesetzt werden. Beispiele für solche Lösungsmittel sind Ether (Dibutylether, Tetrahydrofuran, Dioxan, Ethylenglykoldimethylether, Ethylenglykoldiethylether, lenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), N-alkylierte Carbonsäureamide und Lactame (N,N-Dimethylformamid, N,N-Diethylformamid, N,N-Dimethylacetamid, Tetramethylharnstoff, Hexamethylphosphorsäuretriamid, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), substituierte Benzole (Benzonitril, Chlorbenzol, o-Dichlorbenzol, 1,2,4-Trichlorbenzol, Nitrobenzol), Nitrile (Acetonitril, Propionitril). Geeignet sind auch aromatisch-aliphatische Ether wie zum Beispiel Methyl- oder Ethylphenylether, und Ketone wie zum Beispiel Aceton, Methylethylketon, Methylpropylketon, Dipropylketon, Dibutylketon und Methylisobutylketon.

Eine Gruppe bevorzugter Lösungsmittel sind cyclische Ether, N-alkylierte Säureamide und Lactame, Sulfoxide und Sulfone.

Die erhaltenen Reaktionsprodukte können entweder nach bekannten Verfahren isoliert und gereinigt oder auch direkt anschliessend hydrolysiert und/oder in Salze übergeführt werden.

Die Reaktionsprodukte mit $CCl_3$-Gruppen werden vorteilhaft mit wässrigen Alkalimetallbasen, zum Beispiel KOH oder NaOH hydrolisiert, wobei je nach Einstellung des pH-Wertes nach der Reaktion in an sich bekannter Weise die

Säuren oder deren Natrium- beziehungsweise Kaliumsalze isoliert werden können.

Die erfindungsgemässen Ester können ebenfalls wie zuvor beschrieben mit wässrigen Alkalimetallbasen zu den Säuren hydrolysiert beziehungsweise zu den Salzen umgewandelt werden. Erfindungsgemässe Ester, zum Beispiel Benzylester, können auch katalytisch mit Wasserstoff in Gegenwart von Edelmetallkatalysatoren in die gewünschten Carbonsäuren umgewandelt werden. Ferner ist es möglich, die Estergruppe in eine leicht hydrolysierbare Gruppe umzuwandeln, zum Beispiel mit Trimethylbromsilan, und dann erst diese Gruppe hydrolytisch unter Bildung von Carbonsäuregruppen abzuspalten.

Die Herstellung von Salzen aus den erfindungsgemässen Chitosansäuren oder deren Alkalimetallsalzen kann in an sich bekannter Weise durch die Umsetzung mit Metallsalzen von zum Beispiel Mineralsäuren oder Carbonsäuren in wässriger Lösung erfolgen, wobei insbesondere bei Verwendung mehrwertiger Metallsalze die Chitosansäuresalze unlösliche Polyelektrolytgele bilden. Geeignete Metalle sind zuvor erwähnt worden. Geeignete Metallsalze sind zum Beispiel Oxide, Hydroxide, Fluoride, Chloride, Bromide, Sulfate, Nitrite, Nitrate, Phosphite, Phosphate, Formiate und Acetate.

Die Herstellung von Salzen aus den erfindungsgemässen Chitosansäuren und Ammoniak, Mono- oder Polyaminen oder polymeren Polyaminen kann in der gleichen Weise erfolgen, wobei man auch bei Verwendung von Polyaminen oder polymeren Polyaminen wasserunlösliche Polyelektrolytgele erhält.

Die Isolierung der erfindungsgemässen Chitosanderivate kann nach bekannten Methoden durch zum Beispiel Filtration erfolgen, wobei lösliche Produkte zuvor durch Zugabe von Nichtlösungsmitteln oder durch Einstellung des pH-Wertes ausgefällt werden können. Die Produkte können auch mittels Dialyse oder Leiten über Ionenaustauscherharze isoliert werden. Zur Reinigung können die Produkte gewaschen und anschliessend getrocknet werden, ohne dass hierbei eine vollständige Trocknung erforderlich ist, und die gereinigten Produkte Wassergehalte von bis zu 40 Gew.-% und mehr aufweisen können. Die Produkte können ferner zu Pulvern vermahlen werden. Besonders vorteilhaft ist eine Gefriertrocknung, die zu voluminösen und watteartigen Produkten führt, die besonders leicht löslich und sehr reaktiv sind.

Bei den erfindungsgemässen Chitosanderivaten handelt es um feste Produkte, die in wässrigen oder wässrig-alkalischen Medien oder in polaren organischen Lösungsmitteln gut löslich oder hochquellbar sind. Die freien Säuren oder deren Salze mit nichttoxischen Kationen sind physiologisch verträglich und biologisch abbaubar. Die Produkte eignen sich für die verschiendensten Anwendungen.

Die erfindungsgemässen Chitosanderivate, besonders die Salze und Säuren, sind Polyampholyte mit filmbildenden und sesquestrierenden Eigenschaften auch in Gegenwart von Erdalkalimetallionen. Auf Grund ihrer ausgeprägten sequestrierenden Wirkung für Schwermetallionen bei schon geringen Konzentrationen können die Produkte zur Entfernung solcher Kationen aus kontaminiertem Wasser verwendet werden, zum Beispiel zur Entfernung von Eisen- oder Kupferionen aus Leitungswasser. Ferner können sie als Sesquestriermittel in der Nahrungsmittelindustrie, der pharmazeutischen Industrie und der Textilindustrie eingesetzt werden, sowie auch als Detergentien alleine oder in Kombination mit kationischen, anionischen oder neutralen Detergentien.

Die erfindungsgemässen Chitosanderivate weisen eine überraschend gute komplexierende Wirkung für Metallionen auf, wodurch die Ausfällung von polymeren Metallsalzen, besonders bei mehrwertigen Kationen, günstig beeinflusst oder verhindert werden kann. Ferner zeigen sie eine modulierende Wirkung bei Kristallisationsvorgängen, besonders auf die Bildung von Kristallkeimen, deren Wachstum und die Morphologie der gebildeten Kristalle und deren Grössenverteilung, sowie auf die Aggregations- und Adhäsionseigenschaften. Sie eignen sich daher zur Wasserbehandlung, um zum Beispiel die Abscheidung von Belägen in wasserführenden Systemen (Wasserbehandlungsanlagen), zum Beispiel auf Gefässwänden, Membranen oder Leitungen zu verhindern. Sie können auch zur Vorbehandlung von Textilien, zum Beispiel Baumwolle verwendet werden. Die erfindungsgemässen Chitosanderivate verhindern auch die Bildung von Belägen aus anorganischen und/oder organischen Bestandteilen. Sie eignen sich daher auch als Zusätze in Zahnpflegemitteln gemitteln zur Verhinderung der Bildung von Zahnbelägen, sowie als Zusätze zu Waschmitteln.

Ein weiterer Gegenstand der Erfindung ist ein Verfahren zur Verhinderung von Haftung und/oder Bildung von festen Belägen auf anorganischen oder organischen Substraten, das dadurch gekennzeichnet ist, dass man einer Flüssigkeit oder einem Mittel, das sich in Kontakt mit einem anorganischen oder organischen Substrat befindet, mindestens eines der erfindungsgemässen Chitosanderivate zusetzt, bevorzugt aber 0,01 bis 20 Gew.-%, besonders bevorzugt 0,1 bis 10 Gew.-%.

Die erfindungsgemässen Chitosanderivate haben filmbildende Eigenschaften. Beim Eindampfen von wässrigen Lösungen bilden sich transparente, feste und Wasser enthaltende Filme, die Luft- und Feuchtigkeitsdurchlässig sind. Auf Grund dieser Eigenschaft und ihrer wasserspeichernden Wirkung eignen sie sich auch als Befeuchtigungsmittel für die Haut oder der Schleimhäute in kosmetischen und pharmazeutischen Präparaten, als Mittel zur Aufrechterhaltung der Gelenkbeweglichkeit (Schmiermittelwirkung ähnlich wie Hyaluronsäure), und für Wundverbände. Kosmetische Präparate sind zum Beispiel Haut- und Haarpflegemittel und Deodorantien. Die erfindungsgemässen Chitosanderivate, besonders daraus hergestellte Gele und auch die Ester, eignen sich ferner zur Herstellung von Präparaten mit einer

über einen längeren Zeitraum kontrollierten Wirkstoffabgabe.

Die erfindungsgemässen Chitosanderivate weisen in wässrigen Lösungen auch eine viskositätssteigernde und dispergierende Wirkung auf, wodurch sie sich als Zusätze in Suspensionen, Emulsionen und wässrigen Lösungen eignen, zum Beispiel bei der Herstellung von Lebensmitteln oder Wirkstoffkonzentraten sowie Farbstoff- und Pigmentzubereitungen.

Die erfindungsgemässen Chitosanderivate können auch biocide Wirkungen, zum Beispiel bakteriostatische, fungistatische oder algicide Wirkungen besitzen.

Besonders bevorzugt und ein weiterer Gegenstand der Erfindung ist die Verwendung der erfindungsgemässen Chitosanderivate, besonders der Säuren oder Alkalimetallsalze, zur Verhinderung von Haftung und/oder Bildung von festen Belägen auf anorganischen oder organischen Substraten. Die Beläge, die häufig eine krustenartige Konsistenz besitzen, können sich aus anorganischen und/oder organischen Bestandteilen zusammensetzen, zum Beispiel Salzen und Polymeren auch biologischen Ursprungs. Bei den Substraten kann es sich um anorganische und/oder organische Materialien oder um biologische Materialien handeln, zum Beispiel Glas, Keramiken, Metalle und Metallegierungen, natürliche oder synthetische Kunststoffe, Papier, Textilien, Leder, oder pflanzliche oder tierische Organe oder Gewebe. Ein weiterer Gegenstand der Erfindung ist die Verwendung der erfindungsgemässen Chitosanderivate als Befeuchtigungsmittel der Haut oder der Schleimhäute.

Es wurde ferner überaschend gefunden, dass die erfindungsgemässen Chitosanderivate mit Polyepoxiden vernetzt werden können und hierbei wasserquellbare aber wasserunlösliche Produkte mit guten mechanischen Eigenschaften erhalten werden.

Ein weiterer Gegenstand der Erfindung sind vernetzte Chitosanderivate, erhältlich durch die Umsetzung von erfindungsgemässen Chitosanderivaten mit mindestens einem Polyepoxid, das durchschnittlich mindestens zwei Epoxidgruppen im Molekül enthält.

Als Polyepoxide eignen sich zum Beispiel Glycidylverbindungen mit durchschnittlich zwei Epoxidgruppen im Molekül. Als Glycidylverbindungen kommen vor allem solche mit zwei an ein Heteroatom (z.B. Schwefel, vorzugsweise Sauerstoff oder Stickstoff) gebundenen Glycidylgruppen, β-Methylglycidylgruppen oder 2,3-Epoxycyclopentylgruppen in Frage; genannt seien insbesondere Bis-(2,3-epoxycyclopentyl)-ether; Diglycidylether von mehrwertigen aliphatischen Alkoholen, wie 1,4-Butandiol, oder Polyalkylenglykolen, wie Polypropylenglykole; Diglycidylether von cycloaliphatischen Polyolen, wie 2,2-Bis-(4-hydroxycyclohexyl)-propan; Diglycidylether von mehrwertigen Phenolen, wie Resorcin, Bis-(p-hydroxyphenyl)-methan, 2,2-Bis-(p-hydroxyphenyl)-propan (=Diomethan), 2,2-Bis-(4'-hydroxy-3',5'-dibromphenyl)-propan, 1,3-Di-(p-hydroxyphenyl)-ethan; Di-(β-methylglycidyl)-ether der oben angeführten zweiwertigen Alkohole oder zweiwertigen Phenole; Diglycidylester von Dicarbonsäuren, wie Phthalsäure, Terephthalsäure, $\Delta_4$-Tetrahydrophthalsäure und Hexahydrophthalsäure; N,N-Diglycidylderivate von primären Aminen und Amiden und heterocyclischen, zwei N-Atome enthaltenden Stickstoffbasen, und N,N'-Diglycidylderivate von disekundären Diamiden und Diaminen, wie N,N-Diglycidylanilin, N,N-Diglycidyltoluidin, N,N-Diglycidyl-p-aminophenyl-methyl-ether, N,N'-Dimethyl-N,N'-diglycidyl-bis-(p-aminophenyl)-methan; N',N''-Diglycidyl-N-phenyl-isocyanurat; N,N'-Diglycidylethylenharnstoff; N,N'-Diglycidyl-5,5-dimethyl-hydantoin, N,N'-Diglycidyl-5-isopropyl-hydantoin, N,N-Methylen-bis-(N',N'-diglycidyl-5,5-dimethylhydantoin), 1,3-Bis-(N-glycidyl-5,5-dimethylhydantoin)-2-hydroxypropan; N,N'-Diglycidyl-5,5-dimethyl-6-isopropyl-5,6-dihydro-uracil, Triglycidylisocyanurat.

Bevorzugt sind solche Polyepoxide, die in stark polaren Lösungsmitteln löslich sind, und besonders solche, die in Wasser löslich sind.

Eine bevorzugte Gruppe von Polyepoxiden sind glycidylierte aliphatische Diole und Polyoxaalkylendiole, Novolake, Hydantoine, Aminophenole, Bisphenole und aromatische Diamine oder cycloaliphatische Epoxidverbindungen. Besonders bevorzugte Polyepoxide sind glycidylierte aliphatische Diole und Polyoxaalkylendiole, Kresolnovolake, Bisphenol-A- und Bisphenol-F-diglycidylether, oder Mischungen hiervon.

Insbesondere bevorzugt sind wasserlösliche glycidylierte aliphatische Diole und Polyoxaalkylendiole, da man das Vermischen mit den erfindungsgemässen Chitosanderivaten in einfacher Weise in wässrigen Systemen durchführen kann.

Zur Herstellung der erfindungsgemässen vernetzten Produkte können auch zusätzlich Härtungsbeschleuniger verwendet werden. Beispiele sind 3-Ethyl-4-methylimidazol, Triamylammoniumphenolat); Mono- oder Polyphenole (Phenol, Diomenthan, Salicylsäure); und Phosphorsäure. Härtungsbeschleuniger und Katalysatoren werden üblicherweise in einer Menge von 0,1 bis 10 Gew.-% zugegeben, bezogen auf die Polyepoxidverbindung.

Die Menge des verwendeten Polyepoxids richtet sich hauptsächlich nach dem gewünschten Vernetzungsgrad und den damit verbundenen Eigenschaften. Vorteilhaft sind bis zu 50 %, bevorzugt bis zu 35 % und besonders bevorzugt bis zu 25 % der Strukturelemente der erfindungsgemässen Chitosanderivate vernetzt.

Die Herstellung der vernetzten Derivate kann in an sich bekannter Weise erfolgen, zum Beispiel durch Vermischen der Komponenten gegebenenfalls zusammen mit einem Lösungs- beziehungsweise Suspensionsmittel, das dann mittels Erwärmung entfernt wird. Die Mischung kann dann thermisch vernetzt werden, zum Beispiel durch Erwärmen auf 50 bis 200 °C.

Die vernetzten Chitosanderivate eignen sich besonders zur Herstellung von wasserquellbaren und mechanisch stabilen Formkörpern, wobei die Formgebung mit der Herstellung verbunden werden kann. So kann man Filme und Folien herstellen, die als Membranen oder Wundverbände verwendbar sind, oder man kann Kapseln oder Umhüllungen für Wirkstoffe herstellen, wobei die Wirkstoffabgabe an die Umgebung verzögert wird und kontinuierlich erfolgt.

Die nachfolgenden Beispiele erläutern die Erfindung näher.

A) Herstellung von Zwischenprodukten

Beispiel A1: Umsetzung eines Chitosans mit R(-)-4-Trichlormethyl-2-oxetanon.

a) Aktivierung des Chitosans

Zur Aktivierung wird ein käufliches Produkt (Fluka: mittleres Molekulargewicht $\overline{M}$ ~ 75 000, Acetylgruppengehalt 4,5 %) in 5%iger Essigsäure gelöst, von ungelösten Anteilen abfiltriert, und aus dem Filtrat durch Versetzen mit 2N Natronlauge bis zu einem pH-Wert von 8-9 wieder ausgefällt. Das gequollene weisse Produkt wird durch mehrmaliges Zentrifugieren, Abdekantieren und Wiederaufschlämmen mit destilliertem Wasser oder durch Dialyse der wässrigen Suspension von Salzen befreit. Anschliessend wird das Produkt durch wiederholtes Zentrifugieren mit Dioxan bis zu einem Wassergehalt von 9,8 % entwässert. Eine lyophilisierte Probe dieses Materials ergibt bei der Titration mit 0,1 N HCl unter Berücksichtigung des Wassergehaltes von 9,8 % einen Basengehalt von 5,11 m Val/g. Für die Mehrzahl der nachfolgenden Umsetzungen wird direkt das Dioxan-haltige aktivierte Polymergel eingesetzt.

b) Umsetzung mit R(-)-4-Trichlormethyl-2-oxetanon

546,2 g des Chitosangels (entsprechend 25 g Chitosan = 0,115M) werden in 500 ml trokkenem N-Methylpyrrolidon (NMP) unter Zusatz von 25 g LiCl suspendiert und dann portionenweise bei Raumtemperatur zu einer Lösung von 58,9 g R(-)-4-(Trichlormethyl)-2-oxetanon (0,310 M) in 1 l NMP gegeben. Die entstandene Suspension wird in einem Sulfierkolben mit Rückflusskühler, Flügelrührer und Innenthermometer unter Ausschluss von Luftfeuchtigkeit unter Stickstoffatmosphäre 1 Stunde gerührt, dann auf 55°C aufgeheizt und während 24 Stunden bei dieser Temperatur gehalten. Nach dem Abkühlen wird das erhaltene klare Gel unter starkem Rühren mit 5 Liter Aceton ausgefällt. Die feine Suspension wird über eine Sinternutsche filtriert und mit destilliertem Wasser chloridfrei gewaschen. Das erhaltene Produkt wird nochmals in Methanol suspendiert und erneut filtriert. Eine kleine Probe wird im Vakuum sorgfältig getrocknet und ergibt bei der Elementaranalyse ein Cl/N-Verhältnis von 3,06.

Beispiele A2-A10:

Die in Tabelle 1 aufgeführten Produkte werden gemäss Beispiel A1 hergestellt. In den Beispielen A2 und A5-A8 wird R-, in Beispiel A3 S- und in Beispiel A4 R,S-4-Trichlormethyl-2-oxetanon verwendet und in den Beispielen A9 und A10 wird S-4-Trichlormethyl-4-methyl-2-oxetanon verwendet. Als Lösungsmittel wird Dimethylsulfoxid (DMSO) verwendet (Beispiele A2 bis A4 100 ml, Beispiele A5 bis A7 800 ml, Beispiel A8 400 ml, Beispiel A9 und A10 200 ml). Die Menge an LiCl beträgt 5 g (Beispiele A2 bis A4), 40 g (Beispiele A5 bis A7), 20 g (Beispiel A8), 10 g (Beispiele A9 und A10). Weitere Angaben befinden sich in Tabelle 1. Bei Beispiel A9 beträgt die Reaktionszeit 32 Stunden bei 80°C und bei Beispiel A10 24 Stunden bei 80°C und 24 Stunden bei 100°C. In der letzten Kolonne sind der Gehalt an Cl und N in m Val $CCl_3$/g beziehungsweise m Val N/g und in Klammern das Cl/N-Verhältnis angegeben.

Tabelle 1

| Bsp. Nr. | Chitosan | Menge Oxetanon | Substitutionsgrad (%) | Gehalt Cl/N |
|---|---|---|---|---|
| A2 | 5,0 g=0,031 Mol Sigma; 6,3% Acetyl | 11,7 g=0,062 Mol | 92 | 8,43/2,71 (3,11) |
| A3 | 5,0 g=0,031 Mol Fluka; $\overline{M}$ 2x10$^6$; 4,5% Acetyl | 11,7 g=0,062 Mol | 100 | 9,02/2,57 (3,5) |
| A4 | 5,0 g=0,031 Mol Fluka; $\overline{M}$ 2x10$^6$; 4,5% Acetyl | 11,7 g=0,062 Mol | 100 | 9,22/2,49 (3,7) |
| A5 | 20 g=0,124 Mol Fluka; $\overline{M}$ 2x10$^6$; 4,5% Acetyl | 47,10 g=0,248 Mol | 95 | 8,09/2,48 (3,26) |
| A6 | 20 g=0,124 Mol Fluka; $\overline{M}$ 7,5x10$^5$; 4 % Acetyl | 47,10 g=0,248 Mol | 94 | 8,47/2,50 (3,38) |
| A7 | 20 g=0,124 Mol Fluka; $\overline{M}$ 7x10$^4$; 4,3 % Acetyl | 47,10 g=0,248 Mol | 97 | 8,26/2,49 (3,31) |

Tabelle 1 (fortgesetzt)

| Bsp. Nr. | Chitosan | Menge Oxetanon | Substitutionsgrad (%) | Gehalt Cl/N |
|---|---|---|---|---|
| A8 | 10 g=0,062 Mol Chitosan von Bsp. A6, aktiviert | 23,6 g=0,124 Mol | 100 | 7,95/2,57 (3,01) |
| A9 | 5,0 g=0,031 Mol Fluka;$\overline{M}$ 2x10$^6$; 4,5 % Acetyl | 12,6 g=0,062 Mol | 55 | 6,06/3,24 (1,84) |
| A10 | 5,0 g=0,031 Mol aktiviertes Chitosan nach Bsp. A1, aber aus Dioxan lyophilisiert | 12,6 g=0,062 Mol | 61 | 4,94/2,99 (1,65) |

Beispiel A11:

a) 20 g Chitosan (Fluka, $\overline{M}$ 7,5x10$^5$) wird mit 100 ml HCl (37%) bei 75 °C während 75 Minuten partiell abgebaut zu Chitosan-Oligomeren [A. Domard et. al., Int. J. Biol. Macromol. 11, 297 (1989)]. Das erhaltene Oligomerengemisch wird in wässriger Lösung bis zu einem pH-Wert von 8,5 mit 2N NaOH versetzt und durch Diafiltration über eine Membran mit einer Ausschlussgrenze von 1000 von Salzen und sehr niedrigen molekularen Anteilen befreit. Nach der Gefriertrocknung wird ein Oligomerengemisch mit einem mittleren Polymerisationsgrad von etwa 6-20 erhalten.
b) 0,7 g (4,34x10$^{-3}$ Mol) des getrockneten Oligomerengemisches werden in 15 ml trockenem DMSO zusammen mit 750 mg LiCl suspendiert und anschliessend unter Rühren in eine Lösung von 1,65 g (8,64x10$^{-3}$ M) R,S-4-(Trichlormethyl)-2-oxetanon )-2-oxetanon in 15 ml DMSO, enthaltend 750 mg LiCl, eingetragen. Das Gemisch wird in einer Atmosphäre von trockenem Stickstoff 3 Stunden bei Raumtemperatur und anschliessend 10 Stunden bei 50°C gerührt. Das oligomere Reaktionsprodukt wird mit der 10-fachen Volumenmenge an Dichlormethan ausgefällt. Die Filtration über eine Glasfilternutsche und Nachwaschen mit $CH_2Cl_2$ und Methanol bis zur Chloridfreiheit des Filtrats ergibt nach Trocknen im Vakuum 950 mg eines bräunlichen, pulverigen Produktes, das ein Cl/N-Verhältnis von 1,52 aufweist.

Beispiel A12:

Analog Beispiel A11 werden 150 mg (7,59x10$^{-4}$ Mol) Chitohexaose, die aus kommerziellem Chitohexaosehexahydrochlorid (Ikara Chem. Ind. Tokyo) durch Neutralisieren mit Triethylamin hergestellt wird, in 10 ml trockenem N-Methylpyrrolidon mit 288 mg (1,52x10$^{-3}$ Mol) R(-)-4-Trichlormethyl-2-oxetanon umgesetzt. Es werden 246 mg (Theorie 266 mg) eines beigefarbenen Produktes erhalten, das direkt verseift wird (vergleiche Beispiele B3 und B4).

Beispiel A13:

Analog Beispiel A1 werden 2,0 g (1,24x10$^{-2}$ Mol) eines Chitosan-Produktes, das 50 % Aminogruppen und 50 % Acetylaminogruppen enthält (Chitin 50™, Protan A/S, Norwegen), in N-Methylpyrrolidon (80 ml) unter Zusatz von 4 g LiCl mit 2,36 g (1,24x10$^{-2}$ Mol) R(-)-4-(Trichlormethyl)-2-oxetanon umgesetzt. Zur Vervollständigung der Umsetzung wird das Reaktionsgemisch 18 Stunden bei 80°C und weitere 8 Stunden bei 105°C gerührt. Es werden 1,88 g (59 % der Theorie) eines braunbeigen, pulverigen Produktes erhalten, welches ein Cl/N-Verhältnis von 0,55 aufweist, was einem Substitutionsgrad von 36,6 % entspricht.

Beispiel A14:

Wie in Beispiel 1 beschrieben werden 5 g Chitosan aktiviert und das erhaltene Dioxan-haltige Gel (69,1 g) wird mit 100 ml DMSO, in dem 5 g LiCl gelöst wurden, suspendiert. Die Suspension wird unter $N_2$ in eine Lösung von 14 g (6,2x10$^{-2}$ M) R,S-4-Trichlormethyl-β-sulton [D. Borrmann et. al., Chem. Ber. 99, 1245 (1966)] in 100 ml trockenem DMSO und 5 g LiCl eingetragen und bei Raumtemperatur gerührt. Nach 15 Minuten entsteht ein klares, gelbliches Gel das weitere 12 Stunden bei Raumtemperatur gerührt wird. Anschliessend wird das Reaktionsgemisch in 2 Liter Aceton unter starkem Rühren ausgefällt. Das erhaltene gelbliche Produkt wird zur Reinigung noch zweimal mit frischem Aceton aufgerührt und anschliessend in 200 ml Methanol sowie in 1 Liter Wasser gequollen, um eingeschlossenes LiCl zu entfernen. Das Produkt wird über eine Glassinternutsche filtriert, mit Wasser chloridfrei gewaschen, lyophilisiert und bei 10$^{-4}$ mbar während 24 Stunden getrocknet. Ausbeute: 6,9 g (58 % der Theorie) hellgelbes Polymerpulver, das elementaranalytisch ein Cl/N-Verhältnis von 3,09 m und ein Cl/S-Verhältnis von 3,31 weist.

Beispiel A15:

Analog Beispiel A14 werden 2 g ($1,24x10^{-2}$ Mol) Chitin 50 in 80 ml trockenem DMSO mit 4 g LiCl mit 2,79 g ($1,24x10^{-2}$ Mol) Trichlormethyl-β-sulton umgesetzt. Die erhaltene Reaktionsmischung lässt man 24 Stunden bei 50°C und anschliessend 5 Stunden bei 80°C ausreagieren. Es werden 2,44 g weisses Polymerpulver erhalten, das ein Cl/N-Verhältnis von 1,1 aufweist.

B) Herstellungsbeispiele

Beispiel B1:

Das Produkt gemäss Beispiel A1 wird in methanolfeuchtem Zustand direkt der Verseifung unterworfen. Dazu wird das Produkt bei Raumtemperatur in 750 ml Wasser suspendiert und unter Eiskühlung innerhalb von 45 Minuten mit einer Lösung von 34,1 g NaOH in 254 ml Wasser versetzt. Die entstandene viskose Suspension wird weitere 14 Stunden bei 0-5°C gerührt, anschliessend für weitere 8 Stunden bei Raumtemperatur. Eine Titration des Reaktionsgemisches mit 1N HCl ergibt, dass 3,2 Aequivalente der Base reagiert hatten, der Umsatz also vollständig ist. Die Produktlösung wird mit 2N HCl auf pH 8,0 eingestellt und über eine Glassinternutsche filtriert. Zur Entfernung der enthaltenen anorganischen Salze wird die Lösung anschliessend einer Diafiltration unterworfen, und die chloridfreie Lösung dann lyophilisiert. Das erhaltene weisse, wasserlösliche styroporartige Produkt wird im Hochvakuum getrocknet. Ausbeute 29,9 g entsprechend 69,5 % der Theorie; Wassergehalt 19,11 Gew. %; Carboxylgehalt 3,57 m Val/g (Theorie 3,61).

Beispiel B2:

Zur Herstellung des Natriumsalzes wird eine dreiprozentige wässrige Lösung des gemäss Beispiel B1 erhaltenen Produktes mit der berechneten Menge 2N Natronlauge versetzt, die Lösung dialysiert und anschliessend lyophilisiert. In quantitativer Ausbeute wird ein weisses hochporöses Material erhalten, das sich leicht in Wasser löst.

Beispiel B3:

Die Verseifung des Produktes gemäss Beispiel A12 mit 5 Aequivalenten NaOH in wässriger Suspension gemäss Beispiel B1 ergibt das Natriumsalz des Chitohexaose-hexa-malamids in einer Ausbeute von 135 mg entsprechend 73 % der Theorie, nachdem das Produkt in einem Dialyseschlauch mit einer Ausschlussgrenze von M = 1000 (Spectrapor Nr. 6) von Salzen befreit wird.

Beispiel B4:

Die entsprechende Hexa-carbonsäure aus dem Salz gemäss Beispiel B3 wird hergestellt durch Lösen des erhaltenen Na-Salzes in 5 ml Wasser, Ansäuern der Lösung mit 0,1 N HCl auf pH 3,0 und erneute Dialyse, wie im Beispiel B3 beschrieben. Es werden 93 mg (77,5 % der Theorie) eines leicht gelblichen Pulvers erhalten, das einen Carboxylgruppengehalt von 3,52 m Val/g aufweist.

Beispiel B5:

Analog Beispiel A14 werden 10,0 g (0,062 M) aktiviertes Chitosan (138,2 g Dioxan-haltiges Gel) in 400 ml trokkenem DMSO und 16 g LiCl mit 13,7 g (0,062 M) 4-(Diäthylphosphonomethyl)-2-oxetanon (J.G. Dingwall et. al., J. Chem. Soc., Perkin Trans I 1986, S. 2081) umgesetzt. Die Reaktionsmischung wird 24 Stunden bei 60°C gerührt und dann aufgearbeitet. Es werden 7,5 g eines weissen Polymerpulvers erhalten, das ein P/N-Verhältnis von 0,12 aufweist.

Beispiel B6:

In einem 100 ml Sulfierkolben mit Rührer, Rückflusskühler und Innenthermometer werden in einer Atmosphäre von trockenem Stickstoff 3 g (0,0145 M) 2-Oxetanon-4-carbonsäure-benzylester in 25 ml trockenem Dioxan gelöst In diese Lösung werden bei Raumtemperatur 2,31 g (0,0132 M) aktiviertes Chitosan (nach Beispiel A1; 33,1 g Dioxan-haltiges Chitosan-Gel) eingetragen und während 18 Stunden gerührt. Anschliessend wird 8 Stunden auf 60°C und weitere 48 Stunden auf 100°C erwärmt. Der Verbrauch an ß-Lakton in der Reaktionsmischung als Indiz für das Fortschreiten der Umsetzung kann durch Dünnschichtchromatographie auf Kieselgel mit Toluol/Essigester 1:1 als Laufmittel verfolgt werden. Das Reaktionsprodukt wird anschliessend durch Ausfällen in 500 ml Diethylether isoliert, filtriert und dreimal mit je 100 ml Diethylether nachgewaschen. Nach Suspendieren in 50 ml Dioxan wird das Produkt lyoph-

ilisiert und bei $10^{-2}$ mbar während 24 Stunden über Phosphorpentoxid getrocknet. Es werden 2,85 g (58,8 % der Theorie) eines weissen pulverförmigen Polymerproduktes erhalten, das nach der Elementaranalyse zu 66,2 % substituiert ist.

Beispiel B7:

Zur Entfernung der Benzyl-Schutzgruppe im Produkt gemäss Beispiel B6 werden 2 g des Produktes in 100 ml trockenem Tetrahydrofuran gelöst und nach Zusatz von 40 mg Palladiumacetat bei 50°C und 50 bar Wasserstoffdruck während 21 Stunden hydriert. Anschliessend wird über eine Glassinternutsche vom Katalysator abfiltriert. Aus dem Filterrückstand wird mit 0,1 N NaOH die polymere Säure extrahiert und mit dem ebenfalls auf pH 8 eingestellten Filtrat vereinigt. Nach dem Dialysieren über eine Membran mit einer Ausschlussgrenze von 1000 und anschliessender Gefriertrocknung werden 1,48 g (83 % der Theorie) weisses Pulver erhalten, das das Natriumsalz der polymeren Säure darstellt. Im $^1$H-NMR-Spektrum der Polymeren in DMSO-$d_6$ können keine Signale der Benzylgruppen mehr festgestellt werden.

Beispiel B8:

Analog Beispiel B1 wird das Produkt gemäss Beispiel A14 mit Natronlauge verseift. Dazu wird eine Suspension von 5 g ($12{,}93 \times 10^{-3}$ M) des Produktes in 5 ml Wasser mit einer Lösung von 2,85 g ($71{,}12 \times 10^{-3}$ M) NaOH in 23 ml Wasser versetzt und bei Raumtemperatur 2 Stunden gerührt Dabei geht die Suspension in eine klare Lösung über, die weitere 12 Stunden bei 0-5°C gerührt wird. Eine Titration der Reaktionsmischung mit 0,1 N HCl ergibt, dass 3,64 Aequivalente Natronlauge verbraucht werden. Zur Aufarbeitung wird die Reaktionsmischung über eine Glassinternutsche von geringen Gelanteilen abfiltriert, mit 2N HCl-Lösung unter Eiskühlen auf pH 3,0 eingestellt und anschliessend dialysiert gegen destilliertes Wasser über eine Membran mit einer Ausschlussgrenze von 1000 Dalton. Das Dialysat wird lyophilisiert und das erhaltene Produkt bei $10^{-2}$ mbar während 24 Stunden getrocknet. Das in 65,3 %iger Ausbeute (2,61 g) erhaltene gelbliche Polymer ergibt bei der Titration mit 0,1 N NaOH einen Carboxylgruppengehalt von 1,67 m Val/g.

Beispiele B9-B17:

Analog Beispiel B1 werden die in Tabelle 2 bezeichneten Produkte mit NaOH verseift und die Säuren isoliert.

Tabelle 2

| Beispiel Nr. | Edukt aus Beispiel Nr. | Carboxylgruppengehalt (m Val/g) |
|---|---|---|
| B9 | A2 | 2,8 |
| B10 | A4 | 3,05 |
| B11 | A3 | 3,1 |
| B12 | A5 | 2,5 |
| B13 | A6 | 3,58 |
| B14 | A7 | 3,36 |
| B15 | A8 | 3,65 (100 % der Theorie) |
| B16 | A9 | 1,73 (50 % der Theorie) |
| B17 | A13 | 0,98 (50 % der Theorie) |

Beispiel B18: Vernetzung von Chitosan-Malamidsäure mit Metallionen beziehungsweise Polyaminen unter Bildung von Hydrogelen.

Eine 0,5%ige wässrige Lösung (1 ml) des gemäss Beispiel B2 erhaltenen Natriumsalzes wird bei Raumtemperatur mit jeweils 1 ml einer 3%igen wässrigen Lösung der folgenden Reagentien versetzt, wobei die Vernetzung durch die Bildung eines unlöslichen Niederschlages in Form eines Hydrogels angezeigt wird:

| Salz | Konsistenz |
|---|---|
| Aluminiumacetat | weisses Hydrogel |
| Eisen (III)-nitrat | gelbes Hydrogel |
| Polyethylenimin | weisses Hydrogel |

(fortgesetzt)

| Salz | Konsistenz |
|---|---|
| Chitosan · $CH_3COOH$ | weisses Hydrogel |
| Albumin (aus Rinderblut) | weisses Hydrogel |

Beispiel B19: Triethanolammoniumsalz der Chitosan-Malamidsäure.

400 mg (0,00144 M) Produkt gemäss Beispiel B1 werden bei Raumtemperatur in 100 ml destilliertem Wasser gelöst und ungelöste Anteile werden über eine Glassinternutsche abfiltriert. Zu der klaren Lösung werden 215 mg (0,00144 M) Triethanolamin zugetropft. Die erhaltene viskose Lösung kann mit einem Rackel auf Glasplatten oder Polyesterfolien in dünner Schicht (1000 µm) aufgebracht und an der Luft eingetrocknet werden. Es bilden sich dünne, glasklare Filme, die auf Glas gut haften, von einer Polyesterunterlage aber leicht abgezogen werden können.

Durch Lyophilisieren der Lösung kann das Triethanolaminsalz der Chitosan-Malamidsäure als weisses, voluminöses, in Wasser gut lösliches Pulver gewonnen werden.

Beispiel B20: Vernetzung von Chitosan-Malamidsäure mit Diepoxiden zu Hydrogelfilmen.

22 ml einer 0,3%igen wässrigen Lösung des Produktes gemäss Beispiel B2 werden bei Raumtemperatur mit einer Lösung von 0,01 ml 1,4-Butandiol-diglycidylether in 1 ml Wasser versetzt. Die erhaltene klare Lösung wird wie im Beispiel B19 beschrieben auf einer Polyesterunterlage zu einem Film vergossen. Durch Eintrocknen entsteht ein klarer Film der durch 3-stündiges Erhitzen auf 100°C vernetzt und gehärtet werden kann zu einem wasserquellbaren aber nicht mehr löslichen Ueberzug.

Beispiel B21: Herstellung eines Polyelektrolytgels.

Aequimolare Mengen einer 0,024 molaren wässrigen Lösung des Produktes gemäss Beispiel B2 und einer 0,062 molaren wässrigen Lösung des Chitosan/Essigsäure Salzes werden unter starkem Rühren gemischt. Es entsteht ein Niederschlag, der abfiltriert, mit Wasser neutral gewaschen und gefriergetrocknet wird. Das erhaltene voluminöse Polyelektrolyt-Pulver wird bei $10^{-2}$ mbar während 24 Stunden getrocknet. Es enthält als Komponenten lediglich Chitosan und Aepfelsäure und weist trotz der scharfen Trocknung einen Restwassergehalt von 14,8 Gew-% auf. In Wasser quillt das Produkt zu einem glasklaren sehr voluminösen Gel, das grosse Wassermengen binden kann.

Beispiel B22:

Durch Vergiessen der gemäss Beispiel B21 verwendeten Lösung zu einem Film und Einlegen dieses Films in die Lösung von Chitosan-Acetat wird ein wasserunlöslicher Polyelektrolyt -Film erhalten. Der erhaltene Film ist glasklar, stark lichtbrechend und enthält die 50- bis 100-fache Volumenmenge an Wasser. Der Film weist eine Hybridstruktur auf, in dem lediglich an der Oberfläche des Primärfilms aus Chitosan-Malamidsäure das Polyelektroly-Gel mit Chitosan als polymerem Gegenion gebildet wird.

Nach dem gleichen Verfahren werden Laminat-Filme der umgekehrten Struktur, nämlich mit Chitosan als Primärfilm und Chitosan-Malamidsäure als polymerem Gegenion hergestellt, die ähnliche Eigenschaften aufweisen.

C) Anwendungsbeispiele:

Beispiel C1: Kristallisation von Natriumchlorid unter dem Einfluss von sauren Polysacchariden

Eine Lösung von 204 g Natriumchlorid in 600 ml destilliertem Wasser wird durch ein Membranfilter filtriert und auf 6 Bechergläser verteilt, die eine Abdeckung aus Filterpapier und einen Flügelrührer aufwiesen. Die Rührgeschwindigkeit wird auf 30 Umdrehungen pro Minute eingestellt. Anschliessend wird zu jeder der 6 Lösungen 1 ml einer Lösung zugesetzt, die 1 mg eines der nachstehend aufgeführten Polysaccharide enthält. Die Konzentration an Polysaccharid in der Kristallisationslösung beträgt somit 10 ppm. Anschliessend werden die Lösungen 4 Tage bei Raumtemperatur gerührt und danach die gebildeten Kristalle durch Filtration isoliert. Anhand von mikroskopischen und rasterelektronenmikroskopischen Aufnahmen werden Form, Grösse, Grössenverteilung, Agglomeratbildung und Adhäsion der Kristalle beurteilt. Die Resultate sind in Tabelle 3 zusammengefasst.

Tabelle 3

| Polysaccharid | Kristallform und -grösse | Grössenverteilung | Adhäsion |
|---|---|---|---|
| Hyaluronsäure | Würfel verschiedener Grösse, mm-grosse Agglomerate, unregelmässig gewachsen | breit gestreut 5 µ bis 2 mm | angewachsen an Glaswand und Rührer |
| i-Carrageenan | wie oben, Agglomerate neben mm-grossen Würfeln | breit gestreut 5 µ bis 2 mm | angewachsen an Glaswand und Rührer |
| x-Carrageenan | wie oben Agglomerate neben mm-grossen Würfeln | breit gestreut 5 µ bis 2 mm | angewachsen an Glaswand und Rührer |
| Chondroitin-4-sulfat | runde Agglomerate aus vielen Einzelkristallen, Würfelstruktur | weniger heterogen 30-300 µm | angewachsen an Glaswand und Rührer |
| Chitosan-Malamidsäure nach Bsp. B1 | regelmässige gut ausgebildete Zwilings- bis Vierlinngsaggregate von Oktaederstrukturen | einheitlich 50-100 µm | kein Ansetzen an Glasteilen |
| Blindprobe | Würfelagglomerate verschiedener Grösse | breit gestreut 5 µ bis 2 mm | angewachsen an Glaswand und Rührer |

Beispiel C2:

Kristallisation von Calciumcarbonat unter dem Einfluss von Polysacchariden Durch Suspendieren von 700 mg $CaCO_3$ in 700 ml destilliertem Wasser, Einleiten von $CO_2$ bis zur beinahe vollständigen Lösung und Filtrieren wird eine gesättigte Lösung von Calciumhydrogencarbonat hergestellt. Nach Abfiltrieren der ungelösten Anteile wird die Lösung auf 6 Bechergläser verteilt und wie im Beispiel C1 beschrieben mit jeweils 10 ppm von einem der nachstehend aufgeführten Polysaccharide versetzt. Anschliessend werden die Lösungen 1 Stunde auf 80-85°C erhitzt und die gebildeten $CaCO_3$-Niederschläge wie in Beispiel C1 beschrieben beurteilt.

| Polysaccharid | Kristallform und -grösse | Grössenverteilung | Adhäsion |
|---|---|---|---|
| Blindprobe | Aggregate aus Nadeln | 30-300 µm | zum Teil harte Kruste an Glasteilen |
| Hyaluronsäure | Aggregate aus Nadeln | 30-300 µm | zum Teil harte Kruste an Glasteilen |
| i-Carrageenan | Aggregate aus Nadeln | 30-300 µm | zum Teil harte Kruste an Glasteilen |
| x-Carrageenan | Aggregate aus Nadeln | 30-300 µm | zum Teil harte Kruste an Glasteilen |
| Chondroitin-4-sulfat | verwachsene Nadeln, Aggregate | 30-300 µm | zum Teil harte Kruste an Glasteilen |
| Chitosan-Malamidsäure nach Bsp. C1 | Amorphe Platten 5-10 µm dick | 30-100 µm | Keine Kruste, alles in Suspension |

Beispiel C3:

a) Analog Beispiel C1 werden Lösungen hergestellt, die folgende Ionenkonzentrationen enthalten:

| | | | |
|---|---|---|---|
| 375 ppm | Ca | 220 ppm | $HCO^{\ominus}$ |
| 183 ppm | Mg | 85 ppm | $CO_3^{2\ominus}$ |

Nach Zusatz von 2 bzw. 8 ppm eines Polysaccharids werden die Lösungen 30 Minuten auf 70°C erhitzt und nach

dem Abkühlen filtriert. In den Filtraten wird die nicht auskristallisierte Calciumkonzentration durch Atomabsorption (bzw. durch Titration mit 0,01 M Ethylendiaminteraessigsäure-Lösung) bestimmt und in Prozent Inhibierung ausgedrückt.

b) In analoger Weise wird gemäss dem standardisierten Downell Test die Inhibierung der Bariumsulfat-Kristallisation mit verschiedenen Polysacchariden bestimmt.

Dazu geht man von einer Lösung aus, die 98,9 ppm $Ba^{2+}$, 20 000 ppm NaCl und 8 ppm eines Polysaccharids enthält. Die Lösung wird mit Acetatpuffer auf pH 5,5 eingestellt und bei 25°C gehalten. Anschliessend werden 270,5 ppm $SO_4^{2-}$ zugesetzt, nach 4 Stunden vom ausgefallenen $BaSO_4$ abfiltriert und wie unter a) beschrieben die verbliebene $Ba^{2+}$-Konzentration in den Filtraten bestimmt.

Die erhaltenen Resultate der Kristallisationsinhibierung sind in der Tabelle 4 zusammengefasst.

Tabelle 4

| Polysaccharid | Konzentration ppm | % Kristallisationsinhibierung | |
|---|---|---|---|
| | | $Ca/MgCO_3$ | $BaSO_4$ |
| i-Carrageenan | 8 | 10 | 0 |
| Chondroitin-4-sulfat-Na | 8 | 0 | 0 |
| Chondroitin-6-sulfat-Na | 8 | 0 | 0 |
| x-Carrageenan | 8 | 8,6 | 0 |
| Xanthan | 8 | 10,6 | 0 |
| Polygalacturonsäure | 8 | 20 | 0 |
| Alginsäure | 8 | 27,6 | 0 |
| Chitosan-Malamidsäure nach Bsp. B2 | 2 | 14,6 | 24,8 |
| Chitosan-Malamidsäure nach Bsp. B2 | 8 | 43,6 | 67,2 |

**Patentansprüche**

**1.** Polymere Chitosanderivate mit Strukturelementen der Formeln I, II und III in statistischer Verteilung,

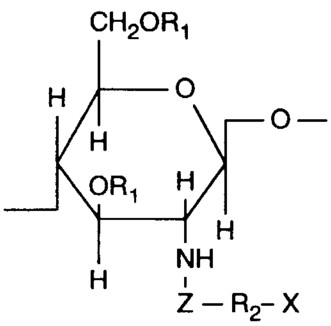
(I)

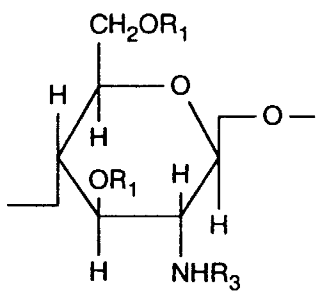
(II)

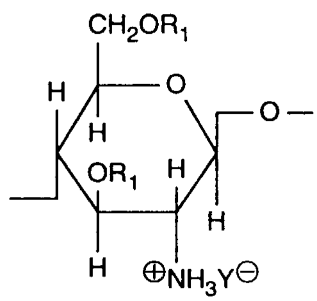
(III),

worin die $R_1$ unabhängig voneinander für H oder den Rest $-Z-R_2-X$ stehen, $R_3$ H oder Acetyl darstellt, Y das Anion $O-Z-R_2-X$ bedeutet, und

(a) Z für -CO- oder $-SO_2-$ steht, X $X-CO_2H$, $-CH_2CO_2H$ oder $-CH_2PO(OH)_2$ bedeutet und $R_2$-$CHR_4CR_5(OH)$-darstellt, $R_4$ -H, -OH, $C_1$-$C_4$-A-koxy oder $C_1$-$C_4$-Alkyl ist, und $R_5$ H oder $C_1$-$C_4$-Alkyl bedeutet, oder

(b) Z für -CO- steht, X $X-CO_2H$ bedeutet und $R_2$-$CHR_6$-$CHR_7$-CH(OH)- oder $-CHR_8-CHR_9-CHR_{10}-CH(OH)$-darstellt, $R_6$, $R_7$, $R_8$ und $R_{10}$ unahängig voneinander -H, -OH, $C_1$-$C_4$-Alkyl oder $C_1$-$C_4$-Alkoxy und $R_9$ -H, -OH, $C_1$-$C_4$-Alkyl, $C_1$-$C_4$-Alkoxy oder $-CO_2H$ bedeuten,

sowie deren Ester und Salze, wobei das Chitosanderivat insgesamt mindestens 2 Strukturelemente enthält und bezogen auf ein Mol des Chitosanderivats 30 bis 100 Mol-% Strukturelemente der Formel I, 60 bis 0 Mol-% Strukturelemente der Formel II und 30 bis 0 Mol-% Strukturelemente der Formel III enthalten sind und sich die Molprozente zu 100 % addieren.

2. Chitosanderivate gemäss Anspruch 1, dadurch gekennzeichnet, dass sie insgesamt mindestens 4 Strukturelemente enthalten.

3. Chitosanderivate gemäss Anspruch 1, dadurch gekennzeichnet, dass sie 4 bis 50 Strukturelemente enthalten.

4. Chitosanderivate gemäss Anspruch 1, dadurch gekennzeichnet, dass sie 6 bis 30 Strukturelemente enthalten.

5. Chitosanderivate gemäss Anspruch 1, dadurch gekennzeichnet, dass sie 50 bis 100 Mol-% Strukturelemente der Formel I, 50 bis 0 Mol-% Strukturelemente der Formel II und 20 bis 0 Mol-% Strukturelemente der Formel III enthalten, wobei sich die Molprozente zu 100 % addieren.

6. Chitosanderivate gemäss Anspruch 5, dadurch gekennzeichnet, dass sie 60 bis 100 Mol-% Strukturelemente der Formel I, 40 bis 0 Mol-% Strukturelemente der Formel II und 10 bis 0 Mol-% Strukturelemente der Formel III enthalten, wobei sich die Molprozente zu 100 % addieren.

7. Chitosanderivate gemäss Anspruch 1, dadurch gekennzeichnet, dass in den Strukturelementen der Formeln I, II und III im Fall (a) Z für -CO- und X für $-CO_2H$ oder $-CH_2PO(OH)_2$ stehen, oder $Z-SO_2-$ und $X-CO_2H$ bedeuten.

8. Chitosanderivate gemäss Anspruch 1, dadurch gekennzeichnet, dass $R_4$ bis $R_{10}$ als Alkyl Methyl, Ethyl, n- oder i-Propyl oder n-, i- oder t-Butyl darstellen.

9. Chitosanderivate gemäss Anspruch 8, dadurch gekennzeichnet, dass $R_4$ bis $R_{10}$ als Alkyl Methyl oder Ethyl darstellen.

10. Chitosanderivate gemäss Anspruch 1, dadurch gekennzeichnet, dass $R_4$ bis $R_{10}$ als Alkoxy Methoxy, Ethoxy, Propoxy oder Butoxy darstellen.

11. Chitosanderivate gemäss Anspruch 10, dadurch gekennzeichnet, dass $R_4$ bis $R_{10}$ als Alkoxy Methoxy bedeutet.

12. Chitosanderivate gemäss Anspruch 1, dadurch gekennzeichnet, dass $R_4$ und $R_6$ bis $R_{10}$ H, OH, Methyl oder Methoxy und $R_5$ H oder Methyl darstellen

13. Chitosanderivate gemäss Anspruch 1, dadurch gekennzeichnet, dass $R_2$ $-CH_2CH(OH)-$, $-CH_2C(CH_3)(OH)-$, $-CH(OH)CH(OH)-$, $-CH(CH_3)CH(OH)-$, $-CH(OCH_3)CH(OH)-$, $-CH_2CH_2CH(OH)-$, $-CH(OH)CH_2CH(OH)-$, $-CH(OH)CH(OH)CH(OH)-$, $-CH(CH_3)CH_2CH(OH)-$, $-CH_2CH(OH)CH(OH)-$, $-CH(OCH_3)CH(OH)CH(OH)-$, $-CH_2CH(OCH_3)CH(OH)-$, $-CH_2CH_2CH_2CH(OH)-$, $-CH_2CH_2CH(OH)CH(OH)-$, $-CH_2CH(OH)CH(OH)CH(OH)-$, $-CH_2CH(COOH)CH_2CH(OH)-$, $-CH_2CH(OH)CH(CH_3)CH(OH)-$ oder $-CH_2CH(OH)CH(OCH_3)CH(OH)-$ bedeutet.

14. Chitosanderivate gemäss Anspruch 13, dadurch gekennzeichnet, dass $R_2$ $-CH_2CH(OH)-$ oder $-CH_2C(CH_3)(OH)-$ darstellt.

15. Chitosanderivate gemäss Anspruch 1, dadurch gekennzeichnet, dass die Carboxyl- und Phosphonsäuregruppen als Ester mit einem aliphatischen, cycloaliphatischen, araliphatischen oder aromatischen Alkohol verestert sind, der 1 bis 30 C-Atome enthält.

16. Chitosanderivate gemäss Anspruch 1, dadurch gekennzeichnet, dass die Carboxyl- beziehungsweise Phosphonsäuregruppen als Salz eines Metalls der Haupt- und Nebengruppen des Periodensystems der Elemente ausgebildet sind.

17. Chitosanderivate gemäss Anspruch 16, dadurch gekennzeichnet, dass die Metalle aus der dritten, vierten und fünften Hauptgruppe und den Nebengruppen des periodischen Systems der chemischen Elemente ausgewählt sind.

18. Chitosanderivate gemäss Anspruch 1, dadurch gekennzeichnet, dass die Carboxyl- beziehungsweise Phosphonsäuregruppen als Ammoniumsalz oder als Salze von primären, sekundären oder tertiären Aminen, als Salz eines

Polyamins mit primären, sekundären oder tertiären Amingruppen, oder als Salz eines Polymeren mit Aminogruppen in wiederkehrenden Strukturelementen ausgebildet sind.

**19.** Verfahren zur Herstellung von Chitosanderivaten gemäss Anspruch 1, dadurch gekennzeichnet, dass man ein Chitosan mit insgesamt mindestens 2 Strukturelementen der Formeln IV und V in statistischer Verteilung,

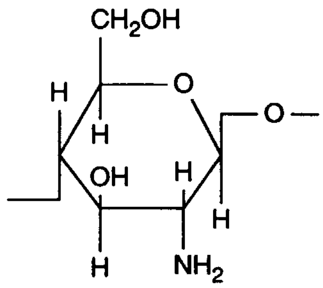 (IV), 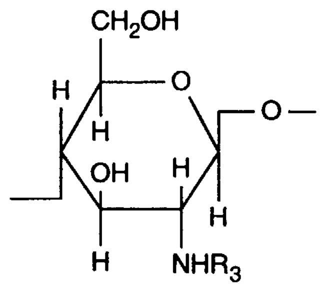 (V),

worin $R_3$ für Acetyl oder H steht, wobei das Chitosan 30 bis 100 Mol-% Strukturelemente der Formel IV und 70 bis 0 Mol-% der Strukturelemente der Formel V enthält, bezogen auf 1 Mol des Chitosans, in Gegenwart eines inerten Lösungsmittels mit mindestens 30 Mol.-% eines Lactons der Formeln VI, VII oder VIII, bezogen auf das Chitosan,

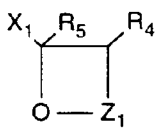 (VI), 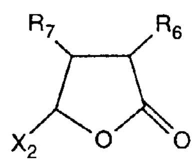 (VII), 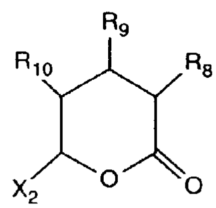 (VIII),

umsetzt, worin $R_4$, $R_5$, $R_6$, $R_7$, $R_8$, $R_9$ und $R_{10}$ die zuvor angegebenen Bedeutungen haben, $Z_1$ für =CO oder $=SO_2$ steht, $X_1$ $-CCl_3$, $-CO_2R_{11}$, $-CH_2CO_2R_{11}$ oder $-CH_2PO(OR_{11})_2$ bedeutet, $X_2$ $-CO_2R_{11}$ darstellt, und $R_{11}$ der um die Hydroxylgruppe verminderte Rest eines Alkohols mit 1 bis 20 C-Atomen bedeutet, und die erhaltenen Chitosanderivate, worin $X_1$ für $-CCl_3$ steht, unter alkalischen Bedingungen unter Bildung der entsprechenden Carbonsäuresalze hydrolysiert, die Carbonsäuresalze und Ester gegebenenfalls in die entsprechenden Säuren überführt und die Säuren gegebenenfalls in Salze überführt.

**20.** Verfahren gemäss Anspruch 19, dadurch gekennzeichnet, dass die Reaktion bei Temperaturen von 0 bis 150 °C durchgeführt wird.

**21.** Verfahren gemäss Anspruch 20, dadurch gekennzeichnet, dass die Reaktion bei Temperaturen von 10 bis 120 °C durchgeführt wird.

**22.** Verfahren gemäss Anspruch 20, dadurch gekennzeichnet, dass die Reaktion bei Temperaturen von 20 bis 100 °C durchgeführt wird.

**23.** Verfahren gemäss Anspruch 19, dadurch gekennzeichnet, dass als Lösungsmittel cyclische Ether, N-alkylierte Säureamide und Lactame, Sulfoxide und Sulfone verwendet.

**24.** Verfahren gemäss Anspruch 19, dadurch gekennzeichnet, dass man die Reaktionsprodukte mit $CCl_3$-Gruppen mit wässrigen Alkalimetallbasen hydrolysiert.

**25.** Verfahren gemäss Anspruch 19, dadurch gekennzeichnet, dass man die Ester mit Alkalimetallbasen zu den Säuren hydrolysiert oder Benzylester zu Säuren hydriert.

**26.** Verfahren gemäss Anspruch 19, dadurch gekennzeichnet, dass man die Säuren mit Metallsalzen, Ammoniak, Mono- oder Polyaminen oder polymeren Aminen in Salze umwandelt.

**27.** Die als Zwischenprodukte verwendeten Chitosanderivate mit Strukturelementen der Formeln IX und X in statistischer Verteilung,

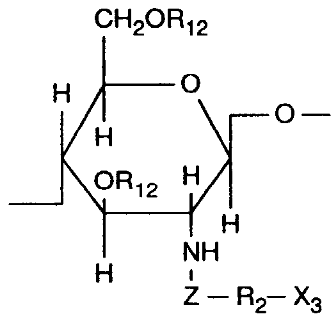

(IX)

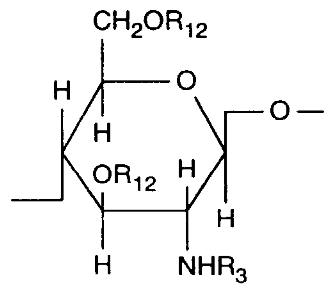

(X)

worin die $R_{12}$ unabhängig voneinander für H oder den Rest $-Z-R_2-X_3$ stehen, $R_3$ H oder Acetyl darstellt, Z für -CO- oder $-SO_2-$ steht, $X_3$-$CCl_3$ bedeutet und $R_2$ $-CHR_4CR_5(OH)-$darstellt, $R_4$ -H, -OH, $C_1$-$C_4$-Alkoxy oder $C_1$-$C_4$-ABkyist, und $R_5$ H oder $C_1$-$C_4$-Alkyl bedeutet, wobei das Chitosanderivat insgesamt mindestens 2 Strukturelemente enthält und bezogen auf 1 Mol des Chitosanderivats 30 bis 100 Mol.-% Strukturelemente der Formel IX und 70 bis 0 Mol.-% Strukturelemente der Formel X enthält.

**28.** Chitosanderivate gemäss Anspruch 27, dadurch gekennzeichnet, dass $R_{12}$ für H steht.

**29.** Chitosanderivate gemäss Anspruch 27, dadurch gekennzeichnet, dass Z für -CO- steht.

**30.** Chitosanderivate gemäss Anspruch 27, dadurch gekennzeichnet, dass $R_2$ $-CH_2CH(OH)-$ oder $CH_2C(CH_3)(OH)-$ bedeutet.

**31.** Chitosanderivate gemäss Anspruch 27, dadurch gekennzeichnet, dass sie insgesamt mindestens 4 Strukturelemente enthalten.

**32.** Chitosanderivate gemäss Anspruch 31, dadurch gekennzeichnet, dass sie 4 bis 50 Strukturelemente enthalten.

**33.** Chitosanderivate gemäss Anspruch 32, dadurch gekennzeichnet, dass sie 6 bis 30 Strukturelemente enthalten.

**34.** Chitosanderivate gemäss Anspruch 27, dadurch gekennzeichnet, dass sie 50 bis 100 Mol-% Strukturelemente der Formel IX und 50 bis 0 Mol-% Strukturelemente der Formel X enthalten.

**35.** Chitosanderivate gemäss Anspruch 34, dadurch gekennzeichnet, dass sie 60 bis 100 Mol-% Strukturelemente der Formel IX und 40 bis 0 Mol-% Strukturelemente der Formel X enthalten.

**36.** Vernetzte Chitosanderivate, erhältlich durch die Umsetzung von Chitosanderivaten gemäss Anspruch 1 mit mindestens einem Polyepoxid, das durchschnittlich mindestens zwei Epoxidgruppen im Molekül enthält.

**37.** Vernetzte Chitosanderivate gemäss Anspruch 36, dadurch gekennzeichnet, dass es sich bei den Polyepoxiden um glycidylierte aliphatische Diole und Polyoxaalkylendiole, Novolake, Hydantoine, Aminophenole, Bisphenole und aromatische Diamine oder cycloaliphatische Epoxidverbindungen handelt.

**38.** Vernetzte Chitosanderivate gemäss Anspruch 36, dadurch gekennzeichnet, dass es sich bei den Polyepoxiden um wasserlösliche glycidylierte aliphatische Diole und Polyoxaalkylendiole handelt.

**39.** Verwendung der Chitosanderivate gemäss Anspruch 1, besonders der Säuren oder Alkalimetallsalze, zur Verhinderung von Haftung und/oder Bildung von festen Belägen auf anorganischen oder organischen Substraten.

**40.** Verwendung der Chitosanderivate gemäss Anspruch 1, besonders der Säuren oder Alkalimetallsalze, als Befeuchtigungsmittel der Haut und von Schleimhäuten.

**Claims**

**1.** A polymeric chitosan derivative with structural units of formulae I, II and III in random distribution

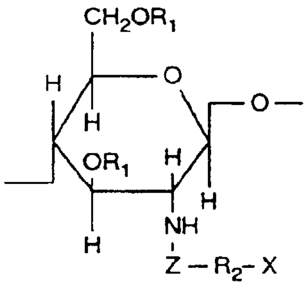
(I)

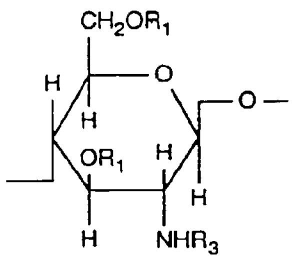
(II)

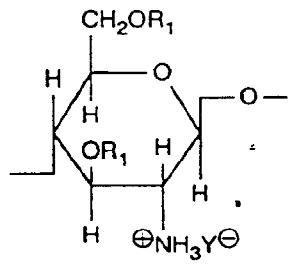
(III),

wherein the substituents $R_1$ are each independently of one another H or the radical $-Z-R_2-X$, $R_3$ is H or acetyl, Y is the anion $O-Z-R_2-X$, and

(a) Z is -CO- or $-SO_2-$, X is $-CO_2H$, $-CH_2CO_2H$ or $-CH_2PO(OH)_2$, and $R_2$ is $-CHR_4CR_5(OH)-$, $R_4$ is -H, $C_1$-$C_4$alkoxy or $C_1$-$C_4$alkyl, and $R_5$ is H or $C_1$-$C_4$alkyl, or
(b) Z is -CO-, X is $-CO_2H$, and $R_2$ is $-CHR_6-CHR_7-CH(OH)-$ or $-CHR_8-CHR_9-CHR_{10}-CH(OH)-$, $R_6$, $R_7$, $R_8$ and $R_{10}$ are each independently of one another -H, -OH, $C_1$-$C_4$alkyl or $C_1$-$C_4$alkoxy, and $R_9$ is -H, -OH, $C_1$-$C_4$alkyl, $C_1$-$C_4$alkoxy or $-CO_2H$,

or an ester or a salt thereof, which chitosan derivative contains a total of at least 2 structural units and, based on one mole of the chitosan derivative, 30 to 100 % molar of structural units of formula I, 60 to 0 % molar of structural units of formula II, and 30 to 0 % molar of structural units of formula III, and the sum of the molar percentages is 100 %.

**2.** A chitosan derivative according to claim 1, which contains a total of at least 4 structural units.

**3.** A chitosan derivative according to claim 1, which contains 4 to 50 structural units.

**4.** A chitosan derivative according to claim 1, which contains 6 to 30 structural units.

**5.** A chitosan derivative according to claim 1, which contains 50 to 100 % molar of structural units of formula I, 50 to 0 % molar of structural units of formula II, and 20 to 0 % molar of structural units of formula III, the sum of the molar percentages being 100 %.

**6.** A chitosan derivative according to claim 5, which contains 60 to 100 % molar of structural units of formula I, 40 to 0 % molar of structural units of formula II, and 10 to 0 % molar of structural units of formula III, the sum of the molar percentages being 100 %.

**7.** A chitosan derivative according to claim 1, wherein in case (a) in the structural units I, II and III, Z is -CO- and X is $-CO_2H$ or $-CH_2PO(OH)_2$, or Z is $-SO_2-$ and X is $-CO_2H$.

**8.** A chitosan derivative according to claim 1, wherein $R_4$ to $R_{10}$ as alkyl are methyl, ethyl, n- or i-propyl or n-, i- or t-butyl.

**9.** A chitosan derivative according to claim 8, wherein $R_4$ to $R_{10}$ as alkyl are methyl or ethyl.

**10.** A chitosan derivative according to claim 1, wherein $R_4$ to $R_{10}$ as alkoxy are methoxy, ethoxy, propoxy or butoxy.

**11.** A chitosan derivative according to claim 10, wherein $R_4$ to $R_{10}$ as alkoxy are methoxy.

**12.** A chitosan derivative according to claim 1, wherein $R_4$ and $R_6$ to $R_{10}$ are H, OH, methyl or methoxy, and $R_5$ is H or methyl.

**13.** A chitosan derivative according to claim 1, wherein $R_2$ is $-CH_2CH(OH)-$, $-CH_2C(CH_3)(OH)-$, $-CH(OH)CH(OH)-$, $-CH(CH_3)CH(OH)-$, $-CH(OCH_3)CH(OH)-$, $-CH_2CH_2CH(OH)-$, $-CH(OH)CH_2CH(OH)-$, $-CH(OH)CH(OH)CH(OH)-$, $-CH(CH_3)CH_2CH(OH)-$, $-CH_2CH(OH)CH(OH)-$, $-CH(OCH_3)CH(OH)CH(OH)-$, $-CH_2CH(OCH_3)CH(OH)-$, $-CH_2CH_2CH_2CH(OH)-$, $-CH_2CH_2CH(OH)CH(OH)-$, $-CH_2CH(OH)CH(oH)CH(OH)-$, $-CH_2CH(COOH)CH_2CH(OH)-$, $-CH_2CH(OH)CH(CH_3)CH(OH)-$or $-CH_2CH(OH)CH(OCH_3)CH(OH)-$.

**14.** A chitosan derivative according to claim 13, wherein $R_2$ is $-CH_2CH(OH)-$ or $-CH_2C(CH_3)(OH)-$.

**15.** A chitosan derivative according to claim 1, wherein the carboxyl and phosphonic acid groups as esters are esterified with an aliphatic, cycloaliphatic, araliphatic or aromatic alcohol which contains 1 to 30 carbon atoms.

**16.** A chitosan derivative according to claim 1, wherein the carboxyl and phosphonic acid groups in salt form are formed from a metal of the main and subsidiary groups of the periodic system of the elements.

**17.** A chitosan derivative according to claim 16, wherein the metals are selected from the third, fourth and fifth main group and the subsidiary groups of the periodic system of the chemical elements.

**18.** A chitosan derivative according to claim 1, wherein the carboxyl and phosphonic acid groups are in the form of ammonium salts or of salts of primary, secondary or tertiary amines, or of a salt of a polyamine containing primary, secondary or tertiary amine groups, or of a salt of a polymer containing amino groups in structural repeating units.

**19.** A process for the preparation of a chitosan derivative according to claim 1, which comprises reacting a chitosan containing a total of at least 2 structural units of formulae IV and V in random distribution

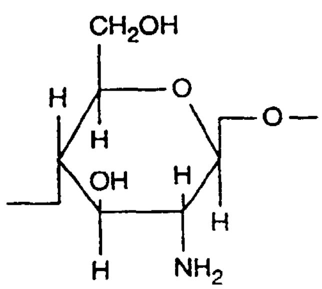
(IV);

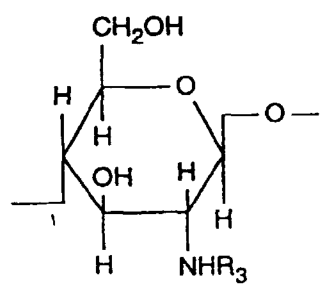
(V),

wherein $R_3$ is acetyl or H, and said chitosan contains 30 to 100 % molar of structural units of formula IV and 70 to 0 % molar of structural units of formula V, based on 1 mol of chitosan, in the presence of an inert solvent, with at least 30 % molar of a lactone of formula VI, VII or VIII, based on said chitosan,

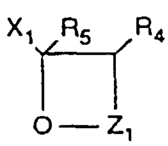
(VI),

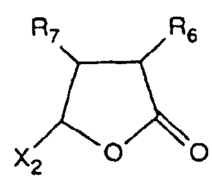
(VII),

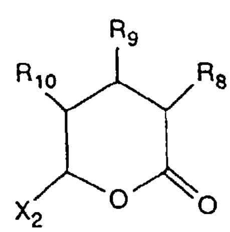
(VIII),

wherein $R_4$, $R_5$, $R_6$, $R_7$, $R_8$, $R_9$ and $R_{10}$ have the meanings previously assigned to them, $Z_1$ is $=CO$ or $=SO_2$, $X_1$ is $-CCl_3$, $-CO_2R_{11}$, $-CH_2CO_2R_{11}$ or $-CH_2PO(OR_{11})_2$, $X_2$ is $-CO_2R_{11}$, and $R_{11}$ is the radical of an alcohol of 1 to 20 carbon atoms which lacks the hydroxyl group, and hydrolysing the resultant chitosan derivative, wherein $X_1$ is $-CCl_3$, under alkaline conditions to form the corresponding carboxylic acid salt, if desired converting the carboxylic acid salt or ester into the corresponding acid and the acid into a salt.

**20.** A process according to claim 19, wherein the reaction is carried out at temperatures from 0 to 150°C.

**21.** A process according to claim 20, wherein the reaction is carried out at temperatures from 10 to 120°C.

**22.** A process according to claim 20, wherein the reaction is carried out at temperatures from 20 to 100°C.

**23.** A process according to claim 19, wherein the solvents used are cyclic ethers, N-alkylated acid amides and lactams, sulfoxides and sulfones.

**24.** A process according to claim 19, wherein the reaction product containing $CCl_3$ groups is hydrolysed with aqueous alkali metal base.

**25.** A process according to claim 19, wherein the ester is hydrolysed with aqueous alkali metal base to the acid or the benzyl ester is hydrogenated to the acid.

**26.** A process according to claim 19, wherein the acid is converted with a metal salt, ammonia, a mono- or polyamine or a polymeric amine into a salt.

**27.** A chitosan derivative used as intermediate, containing structural units of formulae IX and X in random distribution

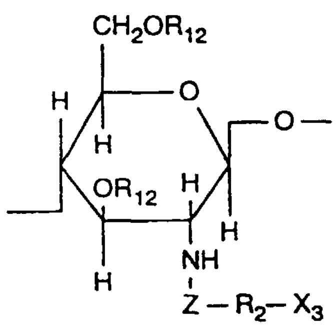

(IX)

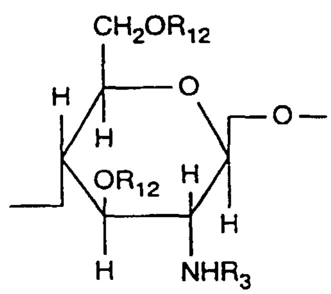

(X)

wherein the substituents $R_{12}$ are each independently of the other H or the radical $-Z-R_2-X_3$, $R_3$ is H or acetyl, Z is -CO- or $-SO_2-$, $X_3$ is $-CCl_3$, and $R_2$ is $-CHR_4,CR_5(OH)-$, $R_4$ is -H, -OH, $C_1$-$C_4$alkoxy or $C_1$-$C_4$alkyl, and $R_5$ is H or $C_1$-$C_4$alkyl, which chitosan derivative contains a total of at least 2 structural units and, based on 1 mol of said chitosan derivative, 30 to 100 % molar of structural units of formula IX and 70 to 0 % molar of structural units of formula X.

**28.** A chitosan derivative according to claim 27, wherein $R_{12}$ is H.

**29.** A chitosan derivative according to claim 27, wherein Z is -CO-.

**30.** A chitosan derivative according to claim 27, wherein $R_2$ is $-CH_2CH(OH)-$ or $CH_2C(CH_3)(OH)-$.

**31.** A chitosan derivative according to claim 27, which contains a total of at least 4 structural units.

**32.** A chitosan derivative according to claim 31, which contains 4 to 50 structural units.

**33.** A chitosan derivative according to claim 32, which contains 6 to 30 structural units.

34. A chitosan derivative according to claim 27, which contains 50 to 100 % molar of structural units of formula IX and 50 to 0 % molar of structural units of formula X.

35. A chitosan derivative according to claim 34, which contains 60 to 100 % molar of structural units of formula IX and 40 to 0 % molar of structural units of formula X.

36. A crosslinked chitosan derivative obtainable by the reaction of a chitosan derivative according to claim 1 with at least one polyepoxide which contains on average at least two epoxy groups in the molecule.

37. A crosslinked chitosan derivative according to claim 36, wherein the polyepoxide comprises glycidylated aliphatic diols and polyoxaalkylenediols, novolaks, hydantoins, aminophenols, bisphenols and aromatic diamines or cycloaliphatic epoxy compounds.

38. A crosslinked chitosan derivative according to claim 36, wherein the polyepoxide comprises water-soluble glycidylated aliphatic diols and polyoxaalkylenediols.

39. Use of a chitosan derivative according to claim 1, especially an acid or alkali metal salt, for the prevention of the adherence to and/or formation of solid deposits on inorganic or organic substrates.

40. Use of a chitosan derivative according to claim 1, especially an acid or alkali metal salt, as humectant for the skin and mucous membranes.

## Revendications

1. Dérivés polymères de chitosane composés de motifs constitutifs de formule (I), (II) et (III) distribués au hasard

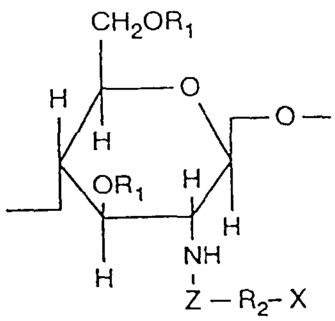
(I)

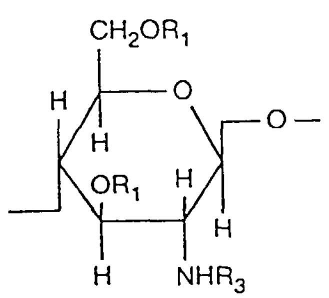
(II)

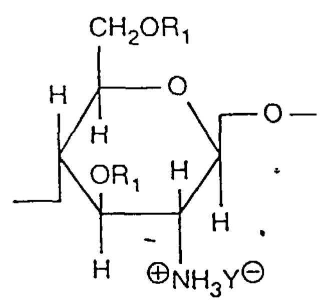
(III),

dans lesquelles les résidus $R_1$ représentent indépendamment un atome d'hydrogène ou le résidu $-Z-R_2-X$, $R_3$ est un atome d'hydrogène ou un groupe acétyle, Y représente l'anion $O-Z-R_2-X$, et a) Z représente un groupe -CO- ou $-SO_2-$, X un groupe $-CO_2H$, $-CH_2CO_2H$ ou $-CH_2PO(OH)_2$ et $R_2$ est un groupe $-CHR_4CR_5(OH)-$, $R_4$ un atome d'hydrogène, un résidu hydroxyle, alcoxy en $C_{1-4}$ ou alkyle en $C_{1-4}$, et $R_5$ est un atome d'hydrogène ou un groupe alkyle en $C_{1-4}$, ou b) Z représente un groupe -CO-, X un groupe $-CO_2H$ et $R_2$ est un groupe $-CHR_6-CHR_7-CH(OH)-$ ou $CHR_8-CHR_9CHR_{10}-CH(OH)$, $R_6$, $R_7$, $R_8$ et $R_{10}$ représentent indépendamment un atome d'hydrogène, un résidu hydroxyle, alkyle en $C_{1-4}$ ou alcoxy en $C_{1-4}$, et $R_9$ est un atome d'hydrogène, un résidu hydroxyle, alkyle en $C_{1-4}$, alcoxy en $C_{1-4}$ ou $-CO_2H$ , ainsi que les esters et sels correspondants, le dérivé de chitosane contenant au total au moins deux motifs constitutifs et, rapporté à une mole de dérivé de chitosane, de 30 à 100 % en moles de motifs constitutifs de formule (I), de 60 à 0 % en moles de motifs constitutifs de formule (II) et de 30 à 0 % en moles de motifs constitutifs de formule (III), les pourcentages en moles se complétant à un total de 100 %.

2. Dérivés de chitosane conformes à la revendication 1, caractérisés en ce qu'ils contiennent au total au moins 4 motifs constitutifs.

3. Dérivés de chitosane conformes à la revendication 1, caractérisés en ce qu'ils contiennent de 4 à 50 motifs constitutifs.

**4.** Dérivés de chitosane conformes à la revendication 1, caractérisé en ce qu'ils contiennent de 6 à 30 motifs constitutifs.

**5.** Dérivés de chitosane conformes à la revendication 1, caractérisés en ce qu'ils contiennent de 50 à 100 % en moles de motifs constitutifs de formule (I), de 50 à 0 % en moles de motifs constitutifs de formule (II) et de 20 à 0 % en moles de motifs constitutifs de formule (III), les pourcentages en moles se complétant à un total de 100 %.

**6.** Dérivés de chitosane conformes à la revendication 5, caractérisés en ce qu'ils contiennent de 60 à 100 % en moles de motifs constitutifs de formule (I), de 40 à 0 % en moles de motifs constitutifs de formule (II) et de 10 à 0 % en moles de motifs constitutifs de formule (III), les pourcentages en moles se complétant à un total de 100 %.

**7.** Dérivés de chitosane conformes à la revendication 1, caractérisés en ce que, dans les motifs constitutifs de formule (I), (II) et (III) dans le cas (a) Z représente un groupe -CO- et X un groupe $-CO_2H$ ou $-CH_2PO(OH)_2$ ou Z représente un groupe-$SO_2$- et X un groupe $-CO_2H$.

**8.** Dérivés de chitosane conformes à la revendication 1, caractérisés en ce que $R_4$ à $R_{10}$, lorsqu'il s'agit de groupes alkyle, représentent des groupes méthyle, éthyle, n- ou i-propyle ou n-, i- ou t-butyle.

**9.** Dérivés de chitosane conformes à la revendication 8, caractérisés en ce que $R_4$ à $R_{10}$, lorsqu'il s'agit de groupes alkyle, représentent des groupes méthyle ou éthyle.

**10.** Dérivés de chitosane conformes à la revendication 1, caractérisés en ce que $R_4$ à $R_{10}$, lorsqu'il s'agit de groupes alcoxy, représentent des groupes méthoxy, éthoxy, propoxy ou butoxy.

**11.** Dérivés de chitosane conformes à la revendication 10, caractérisés en ce que $R_4$ à $R_{10}$, lorsqu'il s'agit de groupes alcoxy, représentent des groupes méthoxy.

**12.** Dérivés de chitosane conformes à la revendication 1, caractérisé en ce que $R_4$ et $R_6$ à $R_{10}$ représentent des résidus H, OH, méthyle ou méthoxy et $R_5$ représente un atome d'hydrogène ou un groupe méthyle.

**13.** Dérivés de chitosane conformes à la revendication 1, caractérisés en ce que $R_2$ représente un résidu $-CH_2CH(OH)-$, $-CH_2C(CH_3C(OH)-$, $-CH(OH)CH(OH)-$, $-CH(CH_3)CH(OH)-$, $-CH(OCH_3)CH(OH)-$, $-CH_2CH_2CH(OH)-$, $-CH(OH)CH_2CH(OH)-$, $-CH(OH)CH(OH)CH(OH)-$, $-CH(CH_3)CH_2CH(OH)-$, $-CH_2CH(OH)CH(OH)-$, $-CH(OCH_3)CH(OH)CH(OH)-$, $-CH_2CH(OCH_3)CH(OH)-$, $-CH_2CH_2CH_2CH(OH)-$, $-CH_2CH_2CH(OH)CH(OH)-$, $-CH_2CH(OH)CH(OH)CH(OH)-$, $-CH_2CH(COOH)CH_2CH(OH)-$, $-CH_2CH(OH)CH(CH_3)CH(OH)--CH_2CH(OH)CH(OCH_3)CH(OH)-$

**14.** Dérivés de chitosane conformes à la revendication 13, caractérisés en ce que $R_2$ représente un résidu $-CH_2CH(OH)-$ ou $-CH_2C(CH_3)(OH)-$.

**15.** Dérivés de chitosane conformes à la revendication 1, caractérisés en ce que les groupes acide carboxylique ou acide phosphonique, lorsqu'ils sont estérifiés, sont estérifiés par un alcool aliphatique, cycloaliphatique, araliphatique ou aromatique comportant de 1 à 30 atomes de carbone.

**16.** Dérivés de chitosane conformes à la revendication 1, caractérisés en ce que les groupes acide carboxylique ou acide phosphonique sous forme de sel, sont des sels d'un métal d'un groupe principal ou secondaire du Tableau Périodique des Eléments Chimiques.

**17.** Dérivés de chitosane conformes à la revendication 16, caractérisés en ce que les métaux sont choisis parmi les métaux du troisième, quatrième et cinquième groupe principal et des groupes secondaires du Tableau Périodique des Eléments Chimiques.

**18.** Dérivés de chitosane conformes à la revendication 1, caractérisés en ce que les groupes acide carboxylique ou acide phosphonique sont sous forme de sel d'ammonium, sous forme de sel d'amines primaires, secondaires ou tertiaires, sous forme de sel d'une polyamine comportant des groupes amine primaires, secondaires ou tertiaires, ou sous forme de sel d'un polymère dont les motifs répétitifs contiennent des groupes amino.

**19.** Procédé pour la préparation de dérivés de chitosane conformes à la revendication 1, caractérisé en ce que l'on fait réagir un chitosane comportant au moins 2 motifs constitutifs de formule (IV) et (V) distribués au hasard

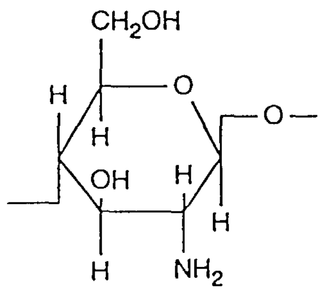 (IV), 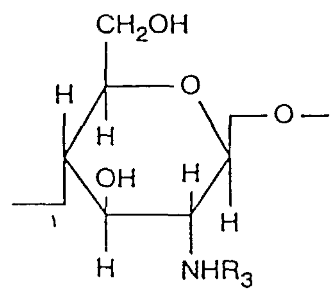 (V),

où $R_3$ représente un groupe acétyle ou un atome d'hydrogène, le chitosane comportant de 30 à 100 % en moles de motifs constitutifs de formule (IV) et de 70 à 0 % en moles de motifs constitutifs de formule (V), rapporté à 1 mole de chitosane,
en présence d'un solvant inerte avec au moins 30 % en moles, rapporté au chitosane, d'une lactone de formule (VI), (VII) ou (VIII),

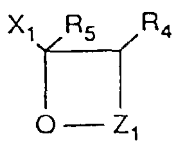 (VI), 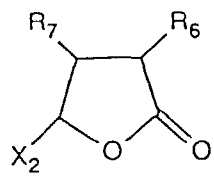 (VII), 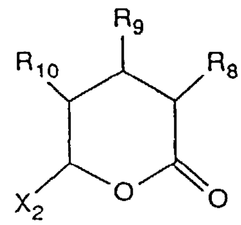 (VIII),

dans lesquelles $R_4$, $R_5$, $R_6$, $R_7$, $R_8$, $R_9$ et $R_{10}$ ont la signification indiquée précédemment, $Z_1$ représente un groupe =CO ou $=SO_2$, $X_1$ représente un groupe $-CCl_3$, $-CO_2R_{11}$, $-CH_2CO_2R_{11}$ ou $-CH_2PO(OR_{11})_2$, $X_2$ est un groupe $-CO_2R_{11}$, et $R_{11}$ est le résidu d'un alcool en $C_{1-20}$ sans la fonction hydroxyle, et en ce que l'on soumet les dérivés de chitosane obtenus, dans lesquels $X_1$ représente un groupe $-CCl_3$, à une hydrolyse alcaline ce qui donne les sels d'acide carboxylique correspondants, en ce que l'on convertit les sels d'acide carboxylique et les esters obtenus éventuellement en acide et les résidus d'acide éventuellement en sels.

**20.** Procédé conforme à la revendication 19, caractérisé en ce que la réaction est réalisée à des températures comprises entre 0 et 150 °C.

**21.** Procédé conforme à la revendication 20, caractérisé en ce que la réaction est réalisée à des températures comprises entre 10 et 120 °C.

**22.** Procédé conforme à la revendication 20, caractérisé en ce que la réaction est réalisée à des températures comprises entre 20 et 100 °C.

**23.** Procédé conforme à la revendication 19, caractérisé en ce que l'on utilise comme solvant, des éthers cycliques, des amides et lactames N-alkylés, des sulfoxydes et des sulfones.

**24.** Procédé conforme à la revendication 19, caractérisé en ce que l'on soumet les produits réactionnels comportant des groupes $CCl_3$ à une hydrolyse par une solution aqueuse d'une base d'un métal alcalin.

**25.** Procédé conforme à la revendication 19, caractérisé en ce que l'on convertit les esters en acides par hydrolyse avec des bases de métaux alcalins ou en ce que l'on convertit les esters de benzyle en acides par hydrogénation.

**26.** Procédé conforme à la revendication 19, caractérisé en ce que l'on convertit les acides en sels par addition de sels métalliques, d'ammoniaque, de mono- ou polyamines ou d'amines polymères.

**27.** Dérivés de chitosane utilisés en tant que produits intermédiaires comportant des motifs constitutifs de formule IX et X distribués au hasard,

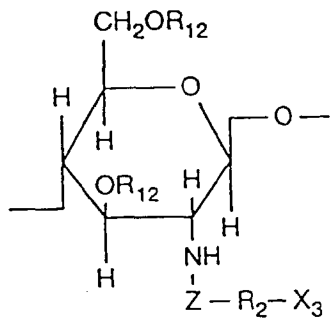
(IX)

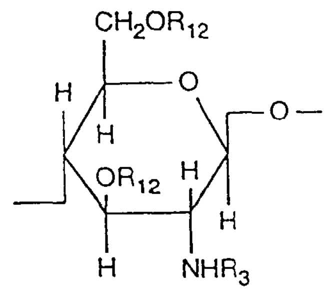
(X)

où $R_{12}$ représente indépendamment un atome d'hydrogène ou le résidu $-Z-R_2-X_3$, $R_3$ représente un atome d'hydrogène ou un résidu acétyle, Z représente un groupe -CO- ou $-SO_2-$, $X_3$ un résidu $CCl_3$ et $R_2$ un résidu $-CHR_4CR_5(OH)-$, R un atome d'hydrogène, un groupe hydroxyle, alcoxy en $C_{1-4}$ ou alkyle en $C_{1-4}$, et $R_5$ un atome d'hydrogène ou un résidu alkyle en $C_{1-4}$, le dérivé de chitosane comportant au total au moins 2 motifs constitutifs et, rapporté à 1 mole de dérivé de chitosane, de 30 à 100 % en mole de motifs constitutifs de formule (IX) et de 70 à 0 % en moles de motifs constitutifs de formule (X).

**28.** Dérivés de chitosane conformes à la revendication 27, caractérisés en ce que $R_{12}$ représente un atome d'hydrogène.

**29.** Dérivés de chitosane conformes à la revendication 27, caractérisés en ce que Z représente un groupe -CO-.

**30.** Dérivés de chitosane conformes à la revendication 27, caractérisés en ce que $R_2$ représente un groupe $-CH_2CH(OH)-$ ou $-CH_2C(CH_3)(OH)-$.

**31.** Dérivés de chitosane conformes à la revendication 27, caractérisés en ce qu'ils contiennent au total au moins 4 motifs constitutifs.

**32.** Dérivés de chitosane conformes à la revendication 31, caractérisés en ce qu'ils contiennent de 4 à 50 motifs constitutifs.

**33.** Dérivés de chitosane conformes à la revendication 32, caracérisés en ce qu'ils contiennent de 6 à 30 motifs constitutifs.

**34.** Dérivés de chitosane conformes à la revendication 27, caractérisés en ce qu'ils contiennent de 50 à 100 % en moles de motifs constitutifs de formule (IX) et de 50 à 0 % en moles de motifs constitutifs de formule (X).

**35.** Dérivés de chitosane conformes à la revendication 34, caractérisés en ce qu'ils contiennent de 60 à 100 % en moles de motifs constitutifs de formule (IX) et de 40 à 0 % en moles de motifs constitutifs de formule (X).

**36.** Dérivés de chitosane réticulés, que l'on peut obtenir par réaction de dérivés de chitosane conformes à la revendication 1 avec au moins un polyépoxyde contenant en moyenne au moins deux groupes époxyde par molécule.

**37.** Dérivés de chitosane réticulés conformes à la revendication 36, caractérisés en ce les polyépoxydes sont des diols aliphatiques et polyoxa-alkylènediols, novolaques, hydantoïnes, aminophénols et diamines aromatiques glycidylés, ou des composés époxyde cycloaliphatiques.

**38.** Dérivés de chitosane réticulés conformes à la revendication 36, caractérisés en ce que les polyépoxydes sont des diols aliphatiques et polyoxa-alkylènediols glycidylés hydrosolubles.

**39.** Utilisation des dérivés de chitosane conformes à la revendication 1, en particulier des acides ou des sels de métaux alcalins, pour éviter l'adhésion et/ou la formation de dépôts solides sur des substrats minéraux ou organiques.

**40.** Utilisation des dérivés de chitosane conformes à la revendication 1, en particulier des acides ou des sels de métaux

alcalins, en tant qu'agent d'humidification de la peau et des muqueuses.